# EUROPEAN PATENT SPECIFICATION

(11) **EP 3 563 818 B1**
(45) Date of publication and mention of the grant of the patent: **12.06.2024**
(21) Application number: 17886450.0
(22) Date of filing: 24.01.2017
(51) Int. Cl.: A61F 13/15, A61F 13/534

(54) **METHOD FOR MANUFACTURING ABSORBENT ARTICLE AND ABSORBENT ARTICLE**
VERFAHREN ZUR HERSTELLUNG EINES SAUGFÄHIGEN ARTIKELS UND SAUGFÄHIGER ARTIKEL
PROCÉDÉ DE FABRICATION D'UN ARTICLE ABSORBANT ET ARTICLE ABSORBANT

(30) Priority: 27.12.2016 CN 201611226900
(43) Date of publication of application: 06.11.2019
(73) Proprietor: Daio Paper Corporation, Ehime 799-0492 (JP)
(72) Inventor: MANABE, Sadanao, Tokyo 102-0071 (JP); OCHI, Ryoichi, Shikokuchuo-shi, Ehime 799-0431 (JP); MIZOBUCHI, Keita, Nantong, Jiangsu 226010 (CN)
(74) Representative: Williams Powell
(86) International application number: PCT/JP2017/002301
(87) International publication number: WO 2018/123071

(56) References cited:
- EP-A1- 2 671 549
- WO-A1-86/05089
- WO-A1-91/09582
- CN-U- 203 341 928
- JP-A- H0 598 521
- JP-A- H06 278 131
- JP-A- H09 266 929
- JP-A- 2006 102 479
- JP-A- 2006 167 196
- JP-A- 2007 144 101
- JP-A- 2008 237 450
- JP-A- 2009 291 475
- US-A1- 2012 034 432

## Description

### Technical Field

The present invention relates to a method for manufacturing an absorbent article including an absorber in which a reinforcing layer such as a sheet is directly sandwiched between accumulation layers obtained by accumulating an accumulation material such as short fibers, and an absorbent article.

### Background Art

Many absorbers of an absorbent article such as a disposable diaper or a sanitary napkin are fiber-stacked bodies obtained by defibrating (also referred to as pulverizing) a short fiber sheet (for example, a pulp sheet obtained by forming pulp fibers into a sheet form) with a defibrating machine, flying the defibrated product on an air flow, mixing super absorbent polymer particles therewith as necessary, and accumulating (fiber accumulating) this mixture in an absorber mold (see Patent Literature 1).

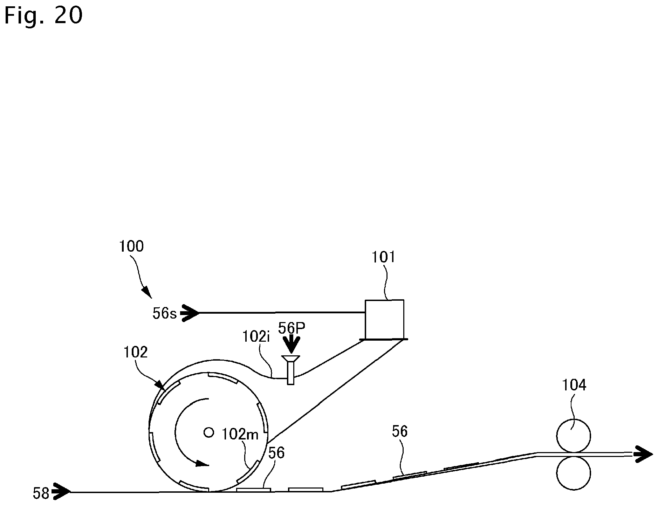

In general, such a fiber accumulating type absorber is manufactured with, for example, manufacturing equipment illustrated in Fig. 20. That is, in manufacturing equipment 100, first, a mixture of pulp fibers obtained by defibrating a pulp sheet 56s with a defibrating machine 101 and super absorbent polymer particles 56P is supplied from above a fiber accumulating drum 102 which is laterally disposed and rotationally driven. On an outer peripheral surface of the fiber accumulating drum 102, an absorber mold 102m, the front-back direction of which is the rotational direction of the accumulating drum 102, is formed in a recessed shape, and many suction holes are formed in a bottom surface of the absorber mold 102m. At a supply position of the pulp fibers and the super absorbent polymer particles 56P, the mixture of the pulp fibers and the super absorbent polymer particles 56P is accumulated in the absorber mold 102m by suction from the suction holes in the absorber mold 102m to form an absorber 56. When the absorber 56 formed in the absorber mold 102m is located so as to face a continuous belt-shaped wrapping sheet 58 supplied along the outer peripheral surface of the fiber accumulating drum 102 by rotation of the fiber accumulating drum 102, the absorber 56 is released from the mold and transferred (moved) onto the wrapping sheet 58. Thereafter, CD (Cross Direction) both side portions of the wrapping sheet onto which the absorber has been transferred are folded back on the absorber 56 at positions along both side edges of the absorber 56 and wrapped by a sailor not illustrated. In a cylindrical continuous body of the wrapping sheet 58 continuous in the MD (Machine Direction), a continuous body of absorbent elements in which the absorbers 56 are intermittently fixed in the MD is formed. The continuous body of the absorbent elements is intermittently cut in the MD in a state where other members are attached during manufacture, in a state where other members are not attached, or in a step of cutting the continuous body into individual finished products to become absorbent elements 50 each having individual absorber.

As the absorber, in addition to an absorber having a single layered structure, an absorber having a plurality of accumulation layers which are different in composition such as the kind of fiber or the content of super absorbent polymer particles is also known. It is known that the absorber having such a plurality of layers is formed by supplying different accumulation materials from a plurality of positions in a rotational direction to a single fiber accumulating drum, or by individually forming each accumulation layer with a plurality of fiber accumulating drums and then making the accumulation layers overlap with one another .

Meanwhile, the present inventor has invented an absorber in which a reinforcing layer such as a sheet is directly sandwiched between a plurality of accumulation layers as a novel structure of an absorber having a plurality of accumulation layers.

However, in a case where an absorber in which a reinforcing layer such as a sheet is directly sandwiched between a plurality of accumulation layers is manufactured, in application of a conventional fiber accumulating drum, a plurality of accumulation layers is sequentially manufactured with a plurality of fiber accumulating drums, a reinforcing layer such as a sheet is stacked on an accumulation layer formed with one fiber accumulating drum, and then a fiber-stacked body formed with the other fiber accumulating drum is disposed on the sheet or the like. Manufacturing equipment is complicated and large, and equipment cost and power consumption cost are high disadvantageously.

### Citation List

### Patent Literature

Patent Literature 1: JP 2015-112393 A
Patent Literature 2: JP 2004-208784 A
Patent Literature 3: US2012/034432
Patent Literature 4: WO86/05089

### Summary of Invention

### Technical Problem

Therefore, a main object of the present invention is to make it possible to manufacture an absorber in which a reinforcing layer such as a sheet is directly sandwiched between a plurality of accumulation layers with simple and compact equipment.

### Solution to Problem

Representative aspects of the present invention solving the above problem are as follows.

### <First Aspect>

A method for manufacturing an absorbent article according to claim 1.

### (Action and effect)

In the present aspect, it is possible to manufacture an absorber in which a reinforcing layer such as a sheet is directly sandwiched between a plurality of accumulation layers with a single fiber accumulating drum. Therefore, manufacturing equipment is simple and compact, and equipment cost and power consumption cost are not high.

### Second Aspect>

The method for manufacturing an absorbent article according to the first aspect, including:
accumulating an accumulation material containing short fibers and not containing super absorbent polymer particles at the first accumulation position; and
accumulating an accumulation material containing short fibers and super absorbent polymer particles at the second accumulation position.

### (Action and effect)

In this way, by accumulating the accumulation material not containing super absorbent polymer particles at the first accumulation position where accumulation is performed first, and accumulating the accumulation material containing super absorbent polymer particles at the second accumulation position after the reinforcing layer is stacked on the first accumulation layer, the super absorbent polymer particles are less likely to reach the suction holes of the absorber mold because the first accumulation layer and the reinforcing layer act as a barrier, and the suction holes of the absorber mold are less likely to be clogged with the super absorbent polymer particles advantageously.

### Third Aspect>

The method for manufacturing an absorbent article according to the second aspect, in which the reinforcing layer is formed of a nonwoven fabric.

### (Action and effect)

Since the nonwoven fabric has fibers intertwined with each other, the nonwoven fabric has excellent shape maintainability and sufficient air permeability as compared with the accumulation layer. Therefore, by sandwiching the nonwoven fabric between the first accumulation layer and the second accumulation layer, the nonwoven fabric functions as a core material, and an absorber has excellent shape maintainability as compared with a case in which only an accumulation layer is included. In addition, since the nonwoven fabric is an assembly of fibers with gaps, the first accumulation layer and the second accumulation layer easily adhere to the nonwoven fabric. In particular, the second accumulation layer is accumulated on the reinforcing layer, and is therefore highly integrated with the reinforcing layer. Furthermore, a super absorbent polymer tries to move from the second accumulation layer to the first accumulation layer due to suction. At this time, some particles of the super absorbent polymer pass through the reinforcing layer formed of the nonwoven fabric to reach the first accumulation layer. However, many particles of the super absorbent polymer are captured by the reinforcing layer formed of the nonwoven fabric, and the amount of polymer in a region in which the reinforcing layer is substantially centrally located in a thickness direction is the largest. A layer with a large amount of polymer tends to reduce strength. However, the substantially centrally located reinforcing layer functions as a core material, and therefore reduction in strength is small even in an absorber with a large amount of polymer.

### Fourth Aspect>

The method for manufacturing an absorbent article according to any one of the first to third aspects, in which
the absorber mold has a narrower portion narrowed on an axial direction central side in an intermediate portion in the rotational direction, and
the width of the reinforcing layer is narrower than the width of the narrowest portion of the narrower portion, and the reinforcing layer is stacked so as to pass through the narrowest portion of the narrower portion.

### (Action and effect)

By supplying the reinforcing layer having the width according to the present aspect in the disposition according to the present aspect, it is possible to form a narrower portion for improving fitting with respect to a periphery of a leg in an intermediate portion in a front-back direction of the first accumulation layer and the second accumulation layer.
Furthermore, in a manufactured absorbent article, the reinforcing layer is not located at the narrower portion of each of the first accumulation layer and the second accumulation layer. Therefore, flexibility of a portion located in the narrower portion is not reduced by the reinforcing layer.

### Fifth Aspect>

An absorbent article as claimed in claim 5.

### (Action and effect)

In general, an absorber of an absorbent article receives forces in various directions from both sides in a width direction due to movement of legs such as walking while being sandwiched between both legs. Therefore, when the absorber is constituted only by an accumulation layer obtained only by accumulating an accumulation material, the absorber easily causes shape collapse such as twisting or cracking disadvantageously.

Meanwhile, when the reinforcing layer is included as in the present aspect, the reinforcing layer functions as a core material, and the absorber has excellent shape maintainability as compared with a case where only the accumulation layer is included. Therefore, the absorber is unlikely to cause shape collapse such as twisting or cracking.

### Sixth Aspect>

The absorbent article according to the fifth aspect, in which the reinforcing layer is formed of a nonwoven fabric or a filament assembly.

### (Action and effect)

Meanwhile, the reinforcing layer formed of a nonwoven fabric or a filament assembly as in the present aspect is preferable because of having excellent air permeability and liquid permeability as well as particularly excellent shape maintainability as compared with the accumulation layer.

### Seventh Aspect>

The absorbent article according to the fifth or sixth aspect, in which
the upper accumulation layer and the lower accumulation layer contain short fibers as an accumulation material,
at least one of the upper accumulation layer and the lower accumulation layer contains super absorbent polymer particles as an accumulation material,
the weight of the super absorbent polymer particles is larger than the weight of fibers contained in the upper accumulation layer and the lower accumulation layer, and
the content of the super absorbent polymer particles stepwise or continuously increases from a back surface side to a front surface side.

### (Action and effect)

In the present aspect, by increasing the content of the super absorbent polymer particles in the absorber and increasing the content of the super absorbent polymer particles in a surface of the absorber, it is possible to effectively suppress returning known as a phenomenon that a liquid component of excrement that has entered the absorber side returns to a skin side. In this way, an absorber having a high content of the super absorbent polymer particles necessarily reduces shape maintainability of the accumulation layer. However, in the present aspect, the above-described reinforcing layer suppresses reduction in shape maintainability.

### Eighth Aspect>

The absorbent article according to any one of the fifth to seventh aspects, in which
each of the upper accumulation layer and the lower accumulation layer has a narrower portion narrowed on a width direction center side in an intermediate portion in a front-back direction, and
the reinforcing layer is disposed within a width direction range of the narrowest portion of the narrower portion.

### (Action and effect)

In the present aspect, since the narrower portion is disposed in a front-back direction intermediate portion in the upper accumulation layer and the lower accumulation layer, fitting with respect to a periphery of a leg is improved. However, since the reinforcing layer is not located at the narrower portion of the upper accumulation layer and the lower accumulation layer, flexibility of a portion located in the narrower portion of the absorbent article is not reduced by the reinforcing layer.

### Ninth Aspect>

The absorbent article according to claim 9.

### (Action and effect)

A layer with a large amount of polymer tends to reduce strength. However, in such a form as in the present aspect, the nonwoven fabric reinforcing layer functions as a core material, and therefore reduction in strength is small even in a portion with a large amount of polymer.

### Advantageous Effects of Invention

As described above, according to the present invention, for example, an absorber in which a reinforcing layer such as a sheet is sandwiched between a plurality of accumulation layers can be manufactured with simple and compact equipment advantageously.

### Brief Description of Drawings

Fig. 1 is a plan view illustrating an inner surface of an underpants-type disposable diaper in an unfolded state.
Fig. 2 is a plan view illustrating an outer surface of an underpants-type disposable diaper in an unfolded state.
Fig. 3 is a cross-sectional view illustrating a layer configuration of a 3-3 cross section of Fig. 1.
Fig. 4 is a cross-sectional view illustrating a layer configuration of a 4-4 cross section of Fig. 1.
Fig. 5 (a) is a cross-sectional view illustrating a layer configuration of a 5-5 cross section of Fig. 1, and Fig. 5(b) is a cross-sectional view illustrating a layer configuration of a 6-6 cross section of Fig. 1.
Fig. 6 is a perspective view of an underpants-type disposable diaper.
Fig. 7 is a cross-sectional view illustrating a layer configuration of a cross section corresponding to a 3-3 cross section of Fig. 1.
Fig. 8 is a cross-sectional view illustrating a layer configuration of a cross section corresponding to a 4-4 cross section of Fig. 1.
Fig. 9 is a plan view illustrating an outer surface of an underpants-type disposable diaper in an unfolded state.
Fig. 10 (a) is a cross-sectional view illustrating a layer configuration of a 5-5 cross section of Fig. 9, and Fig. 10 (b) is a cross-sectional view illustrating a layer configuration of a 6-6 cross section of Fig. 9.
Fig. 11 is a cross-sectional view illustrating a layer configuration of a cross section corresponding to a 3-3 cross section of Fig. 1.
Fig. 12 is a cross-sectional view illustrating a layer configuration of an absorbent element.
Fig. 13 is a cross-sectional view of an absorbent element.
Fig. 14 (a) is a plan view of a main part and Fig. 14 (b) is a schematic longitudinal cross-sectional view, illustrating manufacturing equipment of an absorbent element.
Fig. 15 is a plan view illustrating a process of manufacturing an absorbent element.
Fig. 16 is a cross-sectional view illustrating a process of manufacturing an absorbent element.
Fig. 17 is a schematic longitudinal cross-sectional view illustrating manufacturing equipment of an absorbent element.
Fig. 18 is a schematic longitudinal cross-sectional view illustrating manufacturing equipment of an absorbent element.
Fig. 19(a) is a plan view of a main part and Fig. 19 (b) is a schematic longitudinal cross-sectional view, illustrating manufacturing equipment of an absorbent element.
Fig. 20 is a schematic longitudinal cross-sectional view illustrating manufacturing equipment of an absorbent element.

### Description of Embodiments

Hereinafter, embodiments of the present invention will be described in detail with reference to the attached drawings.

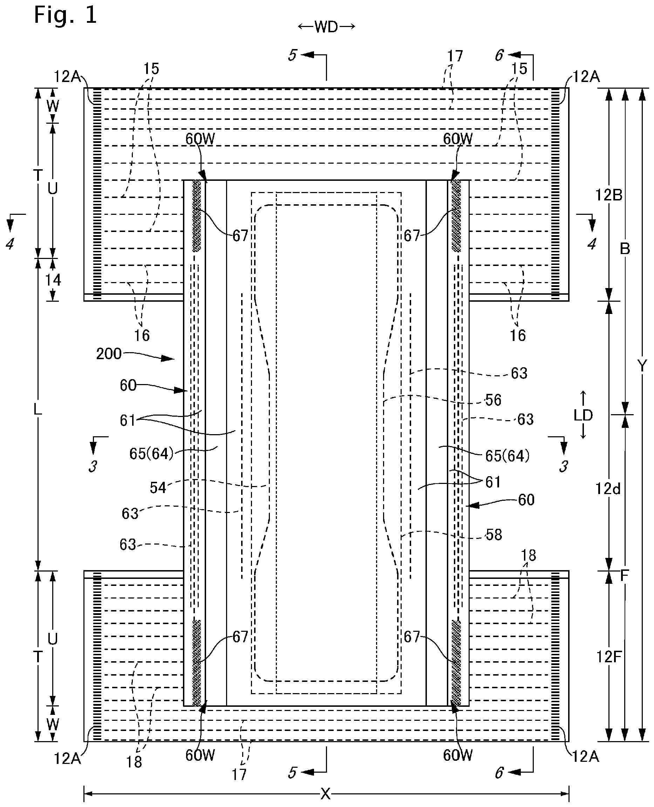

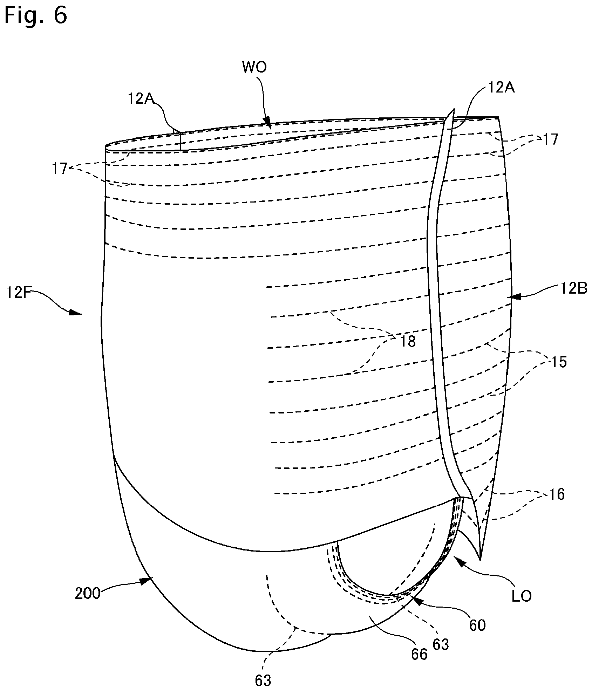

Figs. 1 to 6 illustrate an example of an underpants-type disposable diaper. A dotted pattern portion in the cross-sectional views illustrates an adhesive as a joining means for joining constituent members located on a front surface side and a back surface side, and is formed by applying a hot melt adhesive or the like by solid application, bead application, curtain application, summit application, spiral application, or the like. A fixing portion of an elastically stretchable member is formed, instead of this or in addition to this, by application to an outer peripheral surface of an elastically stretchable member by a comb gun, SureWrap application, or the like. As a joining means for joining constituent members, a fixing means by material welding such as a heat sealing or ultrasonic sealing can also be used.

The underpants-type disposable diaper in the present form includes outer bodies 12F and 12B constituting a front body F and a back body B, and an inner body 200 attached to the outer bodies 12F and 12B so as to extend from the front body F to the back body B through a crotch portion. Both sides of the outer body 12F of the front body F and both sides of the outer body 12B of the back body B are joined to each other to form a side seal portions 12A. A reference numeral Y represents the maximum length of the diaper in an unfolded state (longitudinal length from an edge of a waist opening WO of the front body F to an edge of a waist opening WO of the back body B), and a reference numeral X represents the maximum width of the diaper in an unfolded state.

The inner body 200 is a portion for absorbing and holding excrement such as urine, and the outer bodies 12F and 12B are portions for supporting the inner body 200 with respect to the body of a wearer. In the present embodiment, an upper opening of the outer bodies 12F and 12B is the waist opening WO through which a wearer's torso passes, and a portion surrounded by lower edges of the outer bodies 12F and 12B and a side edge of the inner body 200 at both sides of the inner body 200 in a width direction is a leg opening LO through which a leg passes.

The underpants-type disposable diaper in the present form has a lower torso region T defined as a longitudinal range having the side seal portion 12A (longitudinal range from the waist opening WO to an upper end of the leg opening LO), and an intermediate region L defined as a front-back direction range of a portion forming the leg opening LO (between a longitudinal region having the side seal portion 12A of the front body F and a longitudinal region having the side seal portion 12A of the back body B). The lower torso region T can be divided into a "waist portion" W conceptually forming an edge portion of the waist opening and an "under-waist portion" U which is a portion lower than the waist portion W. In general, in a case where the lower torso region T has a boundary in which a stretching stress in a width direction changes (for example, the thickness of an elastically stretchable member or the stretch rate thereof changes), a portion closer to the waist opening WO than the boundary closest to the waist opening WO is the waist portion W. In a case where there is no such a boundary, a portion closer to the waist opening WO than the absorber 56 or the inner body 200 is the waist portion W. The length thereof in a longitudinal direction varies depending on the size of a product and can be appropriately determined. For example, the length of the waist portion W can be 15 to 40 mm, and the length of the under-waist portion U can be 65 to 120 mm. Meanwhile, both side edges of the intermediate region L are each narrowed in a substantially U shape or a curved shape so as to follow a periphery of a wearer's leg, and the wearer's leg passes therethrough. As a result, the underpants-type disposable diaper in an unfolded state in which the side seal portion 12A has been peeled off has an approximately hourglass shape as a whole.

### (Outer body)

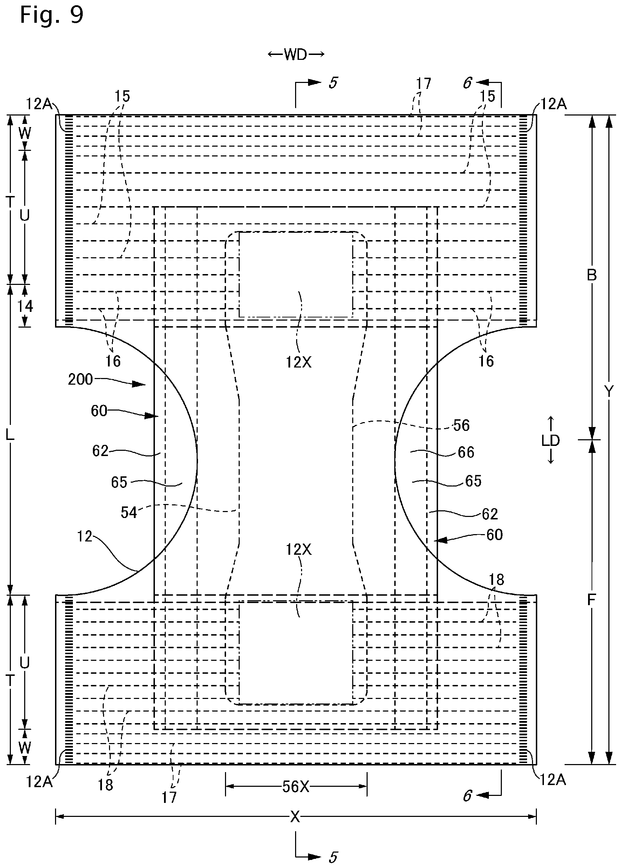

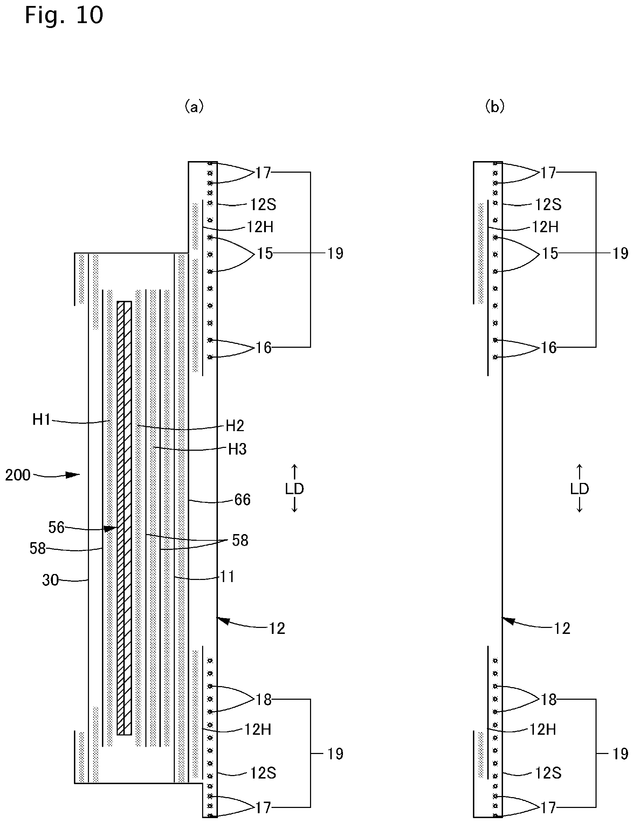

The outer bodies 12F and 12B include the front outer body 12F that is a portion constituting the front body F and the back outer body 12B that is a portion constituting the back body B. The front outer body 12F and the back outer body 12B are not continuous on a leg side but are separated from each other. Its separation distance 12d can be about 150 to 250 mm. Although not illustrated, a crotch portion cover sheet formed of a nonwoven fabric or the like can also be bonded so as to cover a part of an exposed portion of a back surface of the inner body 200 in this separated portion (for example, so as to extend over the entire region in a front-back direction of an exposed portion between the front outer body 12F and the back outer body 12B but not to extend to front and back ends of the inner body 200 and such that both side edges in a width direction do not reach both side edges of the inner body 200) or the whole thereof. As illustrated in Figs. 9 and 10, the outer body 12 can be a continuous integrated body passing through a crotch from the front body F to the back body B. That is, the outer bodies 12F and 12B individually constituting the front body F and the back body B correspond to the former embodiment, and the outer body 12 integrally constituting the front body F and the back body B corresponds to the latter embodiment.

The outer bodies 12F and 12B have a lower torso portion which is a longitudinal range corresponding to the lower torso region T. In the present embodiment, the front outer body 12F does not have a portion corresponding to the intermediate region L, but the back outer body 12B has a gluteal cover portion 14 extending from the lower torso region T to the intermediate region L side. Although not illustrated, in the front outer body 12F, an inguinal cover portion extending from the lower torso region T to the intermediate region L side maybe disposed, or the inguinal cover portion may be disposed without a gluteal cover portion. Alternatively, in both the front outer body 12F and the back outer body 12B, it is not necessary to dispose a portion corresponding to the intermediate region L. In the illustrated form, a lower edge of the gluteal cover portion 14 is formed into a linear shape extending along a width direction similarly to a lower edge of the front outer body 12F, but may be formed into a curved shape so as to be closer to the waist opening side toward the outside in the width direction.

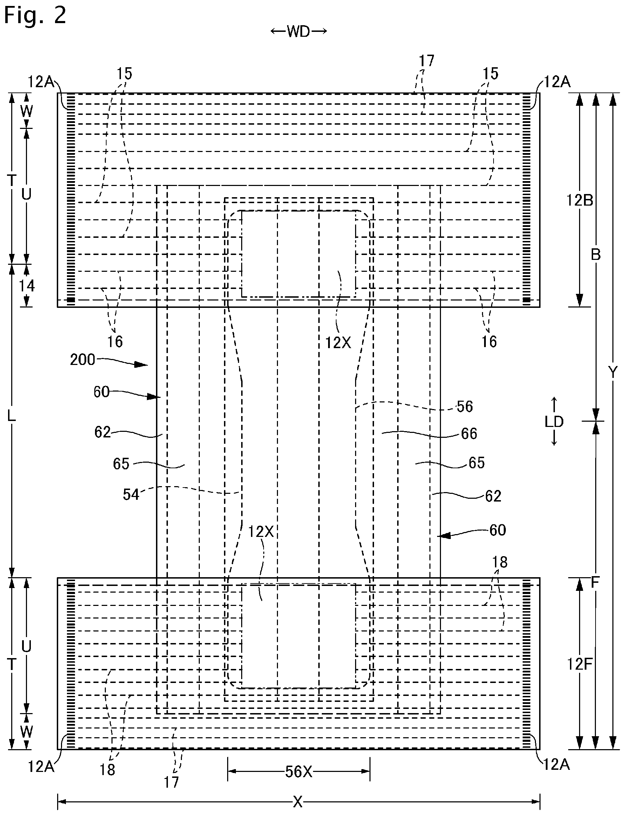

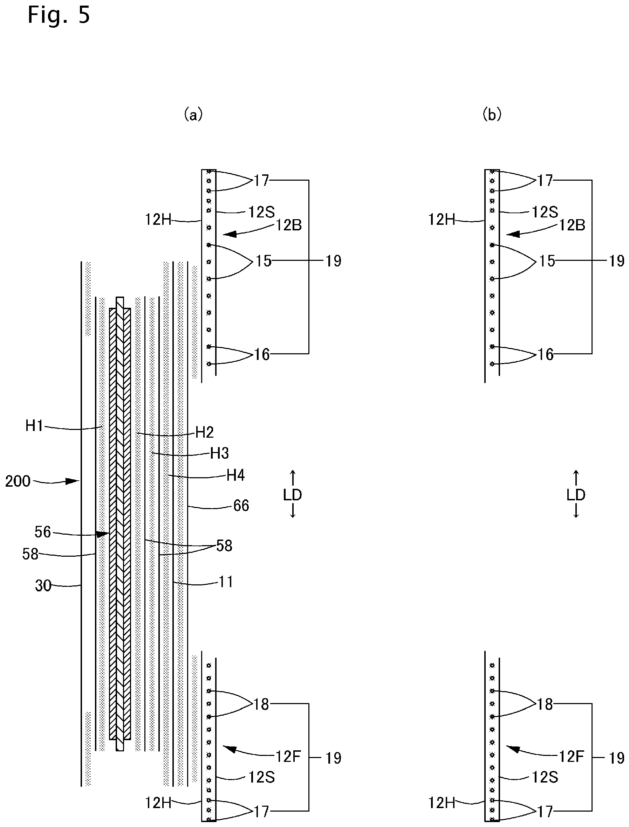

As illustrated in Figs. 2 to 5, the outer bodies 12F and 12B are formed by bonding an outer sheet layer 12S and an inner sheet layer 12H by a bonding means such as a hot melt adhesive or welding. As illustrated in Fig. 5, the outer sheet layer 12S and the inner sheet layer 12H can be formed by folding a single sheet of a sheet material such that a fold is located on the waist opening side. In addition, as illustrated in Fig. 10, the outer sheet layer 12S and the inner sheet layer 12H can be formed by sticking two sheets of a sheet material to each other. In addition, at least one of the outer sheet layer 12S and the inner sheet layer 12H may be formed of a sheet material in which a part thereof is different from the other part.

As the sheet material used for the outer sheet layer 12S and the inner sheet layer 12H, a sheet-shaped material can be used without particular limitation, but a nonwoven fabric is preferable. The nonwoven fabric is not particularly limited concerning a raw material fiber thereof. Examples thereof include a synthetic fiber such as a polyolefin-based fiber including polyethylene and polypropylene, a polyester-based fiber, or a polyamide-based fiber, a regenerated fiber such as rayon or cupra, a natural fiber such as cotton, and a mixed fiber and a composite fiber in which two or more kinds of these fibers are used. Furthermore, the nonwoven fabric may be manufactured by any processing. Examples of a processing method include a known method such as a spunlacing method, a spunbonding method, a thermal bond method, a melt blown method, a needle punching method, an air through method, and a point bond method. In particular, a nonwoven fabric such as an SMS nonwoven fabric or an SMMS nonwoven fabric, in which one or more melt blown nonwoven fabric layers formed of polypropylene ultrafine fiber are sandwiched between spunbonded nonwoven fabric layers formed of polypropylene fiber, is suitable for the outer sheet layer 12S and the inner sheet layer 12H from a viewpoint of flexibility.

In a case where a nonwoven fabric is used, the nonwoven fabric preferably has a fineness of about 0.5 to 2.5 dtex and a basis weight of about 10 to 30 g/m². Therefore, a nonwoven fabric used for the outer bodies 12F and 12B preferably has a total basis weight of about 20 to 60 g/m².

Between the outer sheet layer 12S and the inner sheet layer 12H in the outer bodies 12F and 12B, an elongated elastically stretchable member 19 (15 to 18) such as a rubber thread is disposed at a predetermined stretch rate in a formation region of a stretchable structure in a lower torso direction. As the elongated elastically stretchable member 19, either a synthetic rubber or a natural rubber may be used. For bonding the outer sheet layer 12S and the inner sheet layer 12H in the outer bodies 12F and 12B and fixing the elongated elastically stretchable members 15 to 19 sandwiched therebetween, at least one of a hot melt adhesive by various application methods and a fixing means by material welding such as heat sealing or ultrasonic sealing can be used. When the outer sheet layer 12S and the inner sheet layer 12H are bonded to each other on the entire surface of the outer bodies 12F and 12B, flexibility is impaired. Therefore, the outer sheet layer 12S and the inner sheet layer 12H are preferably bonded to each other intermittently in at least one of a front-back direction and a width direction (for example, the outer sheet layer 12S and the inner sheet layer 12H are not bonded to each other at a passing position of the elongated elastically stretchable member 19, or conversely, the outer sheet layer 12S and the inner sheet layer 12H are bonded to each other only at the passingposition) . Both endportions of the elongated elastically stretchable member 19 in a width direction are fixed to the outer sheet layer 12S and the inner sheet layer 12H (fixed end portions). Preferably, the elongated elastically stretchable member 19 is not fixed to the outer sheet layer 12S and the inner sheet layer 12H between the fixed end portions in terms of flexibility, but may be fixed. The illustrated form is the latter, and the entire elongated elastically stretchable member 19 in a longitudinal direction is fixed to the outer sheet layer 12S and the inner sheet layer 12H. In a case where the elongated elastically stretchable member 19 is fixed to the outer sheet layer 12S and the inner sheet layer 12H with a hot melt adhesive, a method for applying the hot melt adhesive only to an outer peripheral surface of the elongated elastically stretchable member 19 by an application means such as a comb gun or a SureWrap nozzle and sandwiching the elongated elastically stretchable member 19 between the sheet layers 12S and 12H may be used. Alternatively, a method for applying the hot melt adhesive to at least one of the outer sheet layer 12S and the inner sheet layer 12H and sandwiching the elongated elastically stretchable member 19 therebetween may be used.

The illustrated embodiment will be described in more detail. First, between the outer sheet layer 12S and the inner sheet layer 12H in the waist portion W of the outer bodies 12F and 12B, a plurality of waist portion elastically stretchable members 17 is attached at intervals in an up-down direction and in an extended state in a width direction at a predetermined stretch rate so as to be continuous over the entire width direction. One or more waist portion elastically stretchable members 17 disposed in a region adjacent to the under-waist portion U may overlap with the inner body 200, or may be disposed on both sides thereof in a width direction except for a central portion overlapping with the inner body 200 in the width direction. As the waist portion elastically stretchable member 17, it is preferable to attach 3 to 22 rubber threads each having a thickness of 155 to 1880 dtex, particularly about 470 to 1240 dtex (in a case of a synthetic rubber, having a cross section of 0.05 to 1.5 mm², particularly about 0.1 to 1.0 mm² in a case of a natural rubber) at intervals of 4 to 12 mm at a stretch rate of 150 to 400%, particularly about 220 to 320%. All of the waist portion elastically stretchable members 17 do not have to have the same thickness and the same stretch rate. For example, the thickness and the stretch rate of the elastically stretchable member may be different between an upper portion and a lower portion of the waist portion W.

Between the outer sheet layer 12S and the inner sheet layer 12H in the under-waist portion U of the outer bodies 12F and 12B, at each site on an upper side thereof and width direction both sides thereof except for a central portion overlapping with the inner body 200 in the width direction, a plurality of under-waist portion elastically stretchable members 15 and 18 formed of an elongated elastically stretchable member is attached at intervals in an up-down direction and in an extended state in the width direction at a predetermined stretch rate so as to be continuous over the entire width direction.

As the under-waist portion elastically stretchable members 15 and 18, it is preferable to attach 5 to 30 rubber threads each having a thickness of 155 to 1880 dtex, particularly about 470 to 1240 dtex (in a case of a synthetic rubber, having a cross section of 0.05 to 1.5 mm², particularly about 0.1 to 1.0 mm² in a case of a natural rubber) at intervals of 1 to 15 mm, particularly 3 to 8 mm at a stretch rate of 200 to 350%, particularly about 240 to 300%.

Between the outer sheet layer 12S and the inner sheet layer 12H in the gluteal cover portion 14 of the back outer body 12B, at each site on both sides thereof in a width direction except for a central portion overlapping with the inner body 200 in the width direction, a plurality of the cover portion elastically stretchable members 16 formed of an elongated elastically stretchable member is attached at intervals in an up-down direction and in an extended state in a width direction at a predetermined stretch rate so as to be continuous over the entire width direction.

As the cover portion elastically stretchable member 16, it is preferable to attach 2 to 10 rubber threads each having a thickness of 155 to 1880 dtex, particularly about 470 to 1240 dtex (in a case of a synthetic rubber, having a cross section of 0.05 to 1.5 mm², particularly about 0.1 to 1.0 mm² in a case of a natural rubber) at intervals of 5 to 40 mm, particularly 5 to 20 mm at a stretch rate of 150 to 300%, particularly about 180 to 260%.

In a case where an inguinal cover portion is disposed on the front outer body 12F, the cover portion elastically stretchable member can be disposed similarly.

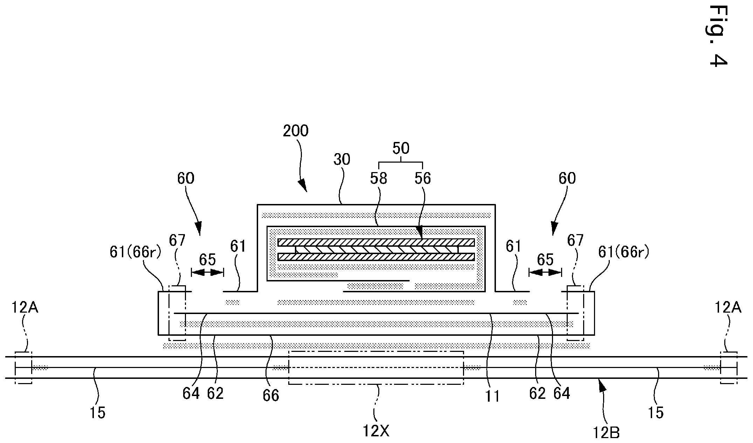

Incidentally, as in the illustrated form, when the elastically stretchable members 19 (the under-waist portion elastically stretchable members 15 and 18 and the cover portion elastically stretchable member 16 in the illustrated embodiment) disposed on the outer bodies 12F and 12B are disposed on both sides thereof in a width direction except for a part or the whole of a portion overlapping with the inner body 200, the inner body 200 is not contracted more than necessary in the width direction, and a poor appearance such as a lumpy appearance is prevented, and absorbability is not reduced. This form includes, in addition to the embodiment in which the elastically stretchable members 19 exist only on both sides in the width direction, an embodiment in which the elastically stretchable member 19 exist across the inner body 200 from one side thereof to the other side thereof in the width direction, but in an intermediate portion in the width direction or the whole in a region overlapping with the inner body 200, as represented by a reference numeral 12X in Figs. 2 and 4, the elastically stretchable member 19 is finely cut, no contraction force acts (substantially equal to a case where no elastically stretchable member is disposed), and only the both sides thereof in the width direction act as contraction force acting portions. Of course, the disposition mode of the elastically stretchable member 19 disposed on the outer bodies 12F and 12B is not limited to the above example. A part or the whole of the elastically stretchable member 19 may be disposed across the inner body 200 from one side thereof to the other side thereof in the width direction such that a stretching force acts on the entire width direction including a portion overlapping with the inner body 200.

### (Inner body)

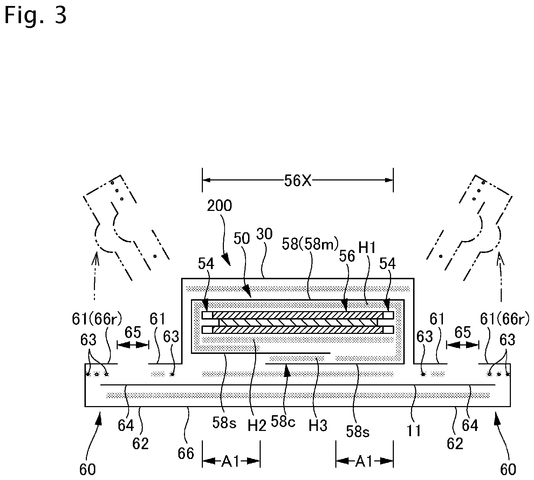

The shape and structure of the inner body 200 are not particularly limited, and for example, shapes and structures described below can be adopted. The inner body 200 can adopt an arbitrary shape, but is rectangular in the illustrated form. As illustrated in Figs. 3 to 5, the inner body 200 includes a liquid pervious top sheet 30 located on a skin side of a wearer, a liquid impervious sheet 11, and the absorbent element 50 interposed therebetween, and is a main unit section having an absorption function. A reference numeral 40 represents an intermediate sheet (also referred to as a second sheet) disposed between the top sheet 30 and the absorbent element 50 in order to rapidly transfer a liquid that has passed through the top sheet 30 to the absorbent element 50. A reference numeral 60 represents an around-leg gather 60 extending along both sides of an absorption surface of the inner body in a width direction and rising toward a periphery of a wearer's leg in order to prevent leakage of excrement into both sides of the inner body 200.

### (Top Sheet)

As the top sheet 30, a liquid pervious material such as a perforated or imperforated nonwoven fabric or a porous plastic sheet can be used without particular limitation. However, in a case where the top sheet 30 also serves as a cover material of a liquid impervious sheet 64 of the around-leg gather 60 as illustrated in Figs. 3 and 4, a nonwoven fabric is used. Among these materials, the nonwoven fabric is not particularly limited concerning a raw material fiber thereof. Examples thereof include a synthetic fiber such as a polyolefin-based fiber including polyethylene and polypropylene, a polyester-based fiber, or a polyamide-based fiber, a regenerated fiber such as rayon or cupra, a natural fiber such as cotton, and a mixed fiber and a composite fiber in which two or more kinds of these fibers are used. Furthermore, the nonwoven fabric may be manufactured by any processing. Examples of a processing method include a known method such as a spunlacing method, a spunbonding method, a thermal bond method, a melt blown method, a needle punching method, an air through method, and a point bond method. For example, if flexibility and drapeability are demanded, a spunbonding method and a spunlacing method are preferable processing methods. If bulkiness and softness are demanded, an air through method, a point bond method, and a thermal bond method are preferable processing methods.

The top sheet 30 may be formed of a single sheet or a stacked sheet obtained by sticking two or more sheets to each other. Similarly, the top sheet 30 may be formed of one sheet or two or more sheets in a plane direction.

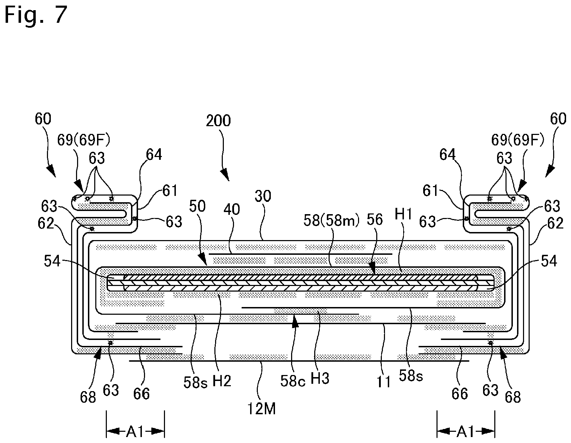

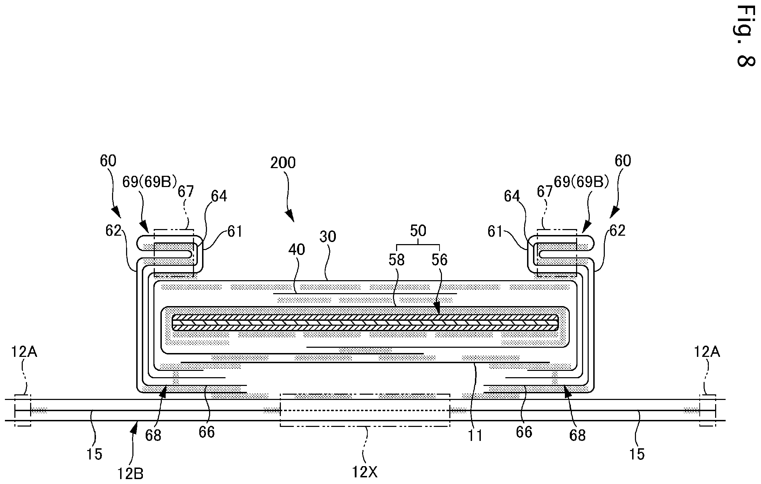

In a case where both sides of the top sheet 30 in a width direction do not also serve as a cover material of the liquid impervious sheet 64 of the around-leg gather 60, for example, as in the embodiment illustrated in Figs. 7 and 8, the top sheet 30 reaches a back surface side of the absorbent element 50 through a portion between the absorbent element 50 and the around-leg gather 60, and can be bonded to the liquid impervious sheet 11 and the around-leg gather 60 with a hot melt adhesive or the like in order to prevent permeation of a liquid.

### (Intermediate sheet)

As in the embodiment illustrated in Figs. 7 and 8, the intermediate sheet (also referred to as "second sheet") 40 that is more hydrophilic than the top sheet can be disposed on a back surface side of the top sheet 30. The intermediate sheet 40 is for preventing a returning phenomenon of an absorbed liquid from an absorber and securing a smooth texture on the top sheet 30. The intermediate sheet 40 can be omitted.

Examples of the intermediate sheet 40 include a similar material to that of the top sheet 30, a spunlaced nonwoven fabric, a spunbonded nonwoven fabric, an SMS nonwoven fabric, a pulp nonwoven fabric, a mixed sheet of pulp and rayon, a point bonded material, and crepe paper. In particular, an air through nonwoven fabric is preferable because of being bulky. As the air through nonwoven fabric, a composite fiber having a core-sheath structure is preferably used. In this case, a resin used for the core may be polypropylene (PP) but is preferably polyester (PET) having high rigidity. The basis weight is preferably 20 to 80 g/m², and more preferably 25 to 60 g/m². A raw material fiber of the nonwoven fabric preferably has a thickness of 2.2 to 10 dtex. In order to make the nonwoven fabric bulky, as mixed fibers of all or some of raw material fibers, eccentric fibers having no core in the center, hollow fibers, eccentric and hollow fibers are also preferably used.

The intermediate sheet 40 in the illustrated embodiment is disposed at the center so as to be shorter than the width of the absorber 56, but may be disposed over the maximum width. The longitudinal length of the intermediate sheet 40 may be the same as the length of the absorber 56, or may be within a short length range centered on a liquid receiving region.

### (Liquid impervious sheet)

A material of the liquid impervious sheet 11 disposed on aback surface side of the absorber 56 is not particularly limited. Examples thereof include a plastic film formed of a polyolefin-based resin such as polyethylene or polypropylene. For the liquid impervious sheet 11, it is preferable to use a liquid impervious and moisture permeable material that has been favorably used from a viewpoint of preventing stuffiness in recent years. As a moisture permeable plastic film, a microporous plastic film obtained by kneading an inorganic filler in an olefin-based resin such as polyethylene or polypropylene, molding a sheet, and then stretching the sheet in a monoaxial or biaxial direction is widely used.

As in the embodiment illustrated in Figs. 3 and 4, the liquid impervious sheet 11 may extend further to a lateral side than the absorber 56 so as to serve also as the liquidpervious film 64 in the around-leg gather 60. Alternatively, as in the embodiment illustrated in Figs. 7 and 8, the liquid impervious sheet 11 may have a width housed in a back surface side of the absorbent element 50, or may go around both sides of the absorbent element 50 in a width direction and extend to both sides of a side surface of the top sheet 30 of the absorbent element 50.

### (Absorbent element)

The absorbent element 50 includes the absorber 56 and the wrapping sheet 58 wrapping the entire absorber 56. The wrapping sheet 58 can also be omitted.

### (Absorber)

The absorber 56 may have a rectangular shape. However, as illustrated in Figs. 1, 3, and the like, the absorber 56 preferably has an hourglass shape having a narrower portion 54 with a narrower width than both sides thereof in a front-back direction in an intermediate portion in the front-back direction because fitting of the absorber 56 and the around-leg gather 60 with respect to a periphery of a leg is improved. The size of the absorber 56 can be determined appropriately. However, the absorber 56 preferably extends to peripheral edges of the inner body 200 or the vicinity thereof in a front-back direction and a width direction. Note that a reference numeral 56x represents the width of the absorber 56.

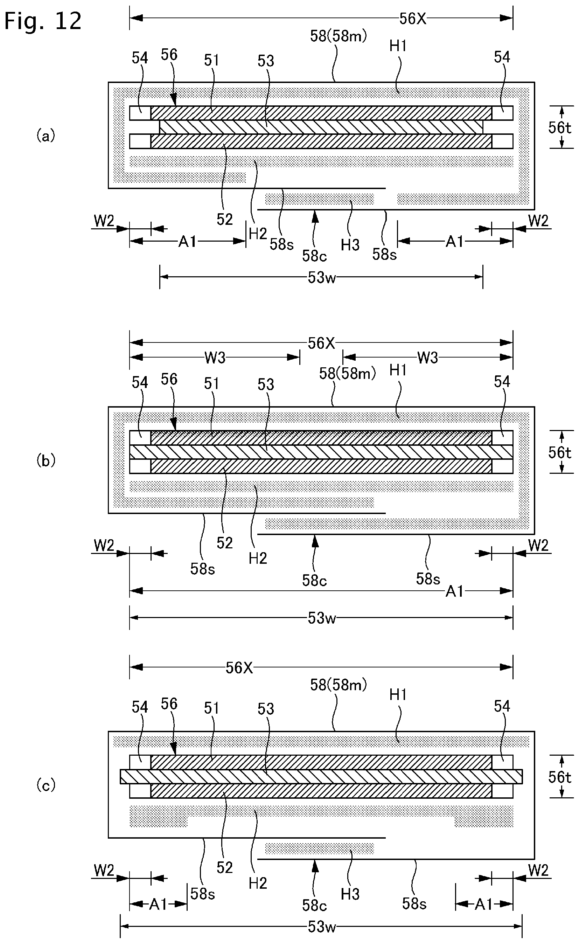

As illustrated in Fig. 12(a), the absorber 56 has a three-layered structure including an upper accumulation layer 51, a lower accumulation layer 52, and a reinforcing layer 53 sandwiched therebetween. The number of layers of the absorber 56 may be four or more. In this case, the absorber 56 only needs to have one or more sets constituted by the reinforcing layer 53 and the upper accumulation layer 51 and the lower accumulation layer 52 sandwiching the reinforcing layer 53. Therefore, for example, the absorber 56 may have a five-layered structure including three accumulation layers in which the reinforcing layer 53 is present between the adjacent accumulation layers. In this case, the layers in contact with the top and bottom of the reinforcing layer 53 are the upper accumulation layer 51 and the lower accumulation layer 52, respectively.

As an accumulation material constituting the upper accumulation layer 51 and the lower accumulation layer 52, short fibers such as pulp fibers (generally 5 mm or less in fiber length) and synthetic fibers (generally about 20 to 80 mm in fiber length) can be used. Super absorbent polymer particles can be mixed and contained partially or entirely in at least one of the upper accumulation layer 51 and the lower accumulation layer 52 as necessary. The total fiber basis weight of the upper accumulation layer and the lower accumulation layer can be, for example, about 100 to 300 g/m². The fineness in a case of the synthetic fibers can be, for example, 1 to 16 dtex, preferably 1 to 10 dtex, and more preferably 1 to 5 dtex.

As the super absorbent polymer particles, those used for this type of absorbent article can be used as they are. The particle diameters of the super absorbent polymer particles are not particularly limited. However, for example, when sieving using a standard sieve of 500 um (JIS Z8801-1: 2006) (shake for five minutes) is performed, and particles falling under the sieve using this sieving are sieved using a standard sieve of 180 um (JIS Z8801-1: 2006) (shake for five minutes), it is desirable that a ratio of particles remaining on the standard sieve of 500 µm is 30% by weight or less, and a ratio of particles remaining on the standard sieve of 180 um is 60% by weight or more.

The water absorption capacity of the super absorbent polymer particles is not particularly limited, but in general, preferably 40 g/g or more. Examples of a material of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

The water absorption rate of the super absorbent polymer particles is not particularly limited, but is usually 70 seconds or less, and particularly preferably 40 seconds or less. When the water absorption rate is too slow, so-called returning that a liquid that has been supplied into the absorber 56 returns out of the absorber 56 tends to occur.

As the super absorbent polymer particles, those having a gel strength of 1000 Pa or more are suitably used. This makes it possible to effectively suppress sticky feeling after liquid absorption even in a case of forming the bulky absorber 56.

The basis weight of the super absorbent polymer particles in the entire absorber 56 can be appropriately determined depending on the absorption amount required for the product. Therefore, for example, the basis weight can be 50 to 350 g/m² although this cannot be applied generally. The basis weight of a polymer of less than 50 g/m² makes it difficult to secure the absorption amount. The basis weight of more than 350 g/m² saturates an effect.

A ratio between the fibers and the super absorbent polymer particles in the whole of the upper accumulation layer 51 and the lower accumulation layer 52 is not particularly limited. However, when a weight ratio of fibers : super absorbent polymer particles is 50 : 50 to 20 : 80, the absorber 56 can be made thinner with the same area and the same absorption amount. In this case, a thickness 56t of the absorber 56 is not particularly limited, but can be 3 to 15 mm.

The content of the super absorbent polymer particles can be changed in a thickness direction of the absorber 56. In particular, when the content (weight percentage) of the super absorbent polymer particles in the absorber 56 increases stepwise or continuously from a back surface side to a front surface side, an excellent property in preventing returning known as a phenomenon that a liquid content of excrement that has entered the absorber 56 side returns to a skin side is obtained. In particular, more preferably, the lower accumulation layer 52 does not contain the super absorbent polymer particles, and only the upper accumulation layer 51 or only the upper accumulation layer 51 and the reinforcing layer 53 contain the super absorbent polymer particles. However, a structure in which the lower accumulation layer 52 simply contains no super absorbent polymer particles reduces the absorption amount and easily causes returning. Therefore, it is desirable that a layer containing super absorbent polymer particles contains more super absorbent polymer particles than a usual layer.

Of course, depending on a purpose, the content of the super absorbent polymer particles 56P in the absorber 56 can be increased stepwise or continuously from a front surface side to a back surface side, or can be increased stepwise or continuously from both the front surface side and the back surface side to an intermediate portion in a thickness direction. For example, a layer having a large amount of polymer tends to reduce strength. However, as described later, in a case where the reinforcing layer 53 is formed of a nonwoven fabric and has better shape maintainability than the accumulation layers 51 and 52, the amount of polymer in a region in which the reinforcing layer 53 is centrally located in a thickness direction is preferably the largest because the reinforcing layer 53 functions as a core material and reduction in strength is therefore small even in a portion where the amount of polymer is large.

Here, the stepwise change in the content of super absorbent polymer particles means a state in which a plurality of layers having different contents is stacked and each layer has a substantially constant content over the entire layer as in a case where the content of super absorbent polymer particles is different among the upper accumulation layer 51, the lower accumulation layer 52, and the reinforcing layer 53 in the absorber 56 illustrated in Fig. 12(a). The continuous change means a state in which the content does not change stepwise at least in the layers 51, 52, and 53 (indicated by gradation in the drawing) as in the absorber 56 illustrated in Fig. 12(b). The "stepwise or continuous increase" in the content of super absorbent polymer particles in the absorber 56 includes, in addition to an embodiment including super absorbent polymer particles in the entire thickness direction of the absorber 56, a form in which a layer containing no super absorbent polymer particles (layer with a content of zero) is included on either a front or a back of the absorber 56, and the content increases from the layer, for example, an embodiment in which only the upper accumulation layer 51 contains super absorbent polymer particles or an embodiment in which only the upper accumulation layer 51 and the reinforcing layer 53 contain super absorbent polymer particles.

In a case where the content of super absorbent polymer particles is changed stepwise, the number of layers having a substantially constant content is not particularly limited, and the thickness of each layer is not particularly limited. For example, as described above, in a case where a thin absorber in which a weight ratio of fibers : super absorbent polymer particles is 50 : 50 to 20 : 80 is formed, and a two-layered structure having a high content layer on a front surface side and a low content layer on a back surface side is formed (for example, the upper accumulation layer 51 is formed of a high content layer and the lower accumulation layer 52 is formed of a low content layer in the embodiment illustrated in Fig. 12 (a)) in order to prevent returning of the absorbed liquid, the content of super absorbent polymer particles in the high content layer is preferably 50 to 90% by weight, and the content of super absorbent polymer particles in the low content layer is preferably 15 to 60% by weight. The thickness of the high content layer is preferably 20 to 60% of the thickness 56t of the absorber 56, and the thickness of the low content layer is preferably 40 to 80% of the thickness 56t of the absorber 56.

The content of the super absorbent polymer particles of the absorber 56 can also be changed in a planar direction if necessary. For example, the content at a liquid excretion site can be larger than that at another site. In a case where a gender difference is considered, the content at a front side can be increased for men, and the content at a central portion can be increased for women. It is also possible to locally dispose a portion where no polymer particles are present (for example, in a spot shape) in a planar direction of the absorber 56.

The reinforcing layer 53 sandwiched between the upper accumulation layer 51 and the lower accumulation layer 52 is not particularly limited, and is preferably air permeable, but may be non-air permeable. The air-permeable reinforcing layer 53 suitably has air permeability of 2 to 60 seconds. The reinforcing layer 53 is preferably hydrophilic but may be water repellent or hydrophobic. Usually, the reinforcing layer 53 preferably has higher shape maintainability than the upper accumulation layer 51 and the lower accumulation layer 52, and preferably has air permeability, hydrophilicity, and a liquid pervious property. Examples of such a reinforcing layer 53 include a nonwoven fabric and a filament assembly. When such a reinforcing layer 53 is sandwiched between the upper accumulation layer 51 and the lower accumulation layer 52, the reinforcing layer 53 functions as a core material, and the absorber 56 has excellent shape maintainability as compared with a case where only the accumulation layer is included. Therefore, the absorber 56 is unlikely to cause shape collapse such as twisting or cracking. In particular, an accumulation layer in which short fibers such as pulp fibers (generally, with a fiber length of 5 mm or less) and super absorbent polymer particles are mixed and accumulated reduces entanglement of fibers by absorption expansion of the super absorbent polymer particles, and particularly easily collapses the shape by an external force. However, such shape collapse can be effectively prevented by a reinforcing effect of the reinforcing layer 53. As the reinforcing layer 53, paper, particularly water-absorbent paper such as kitchen paper or crepe paper, a perforated film having many holes passing through the film from the front to the back, a reticulated material, or the like can be used. In addition, the reinforcing layer 53 may be a fibrous resin layer applied with a hot melt discharge apparatus. As can be understood from this example, the reinforcing layer 53 may have many holes passing through the layer from the front to the back regardless of a material thereof.

A nonwoven fabric used for the reinforcing layer 53 is not particularly limited, but a long fiber nonwoven fabric is suitable. For example, a spunbonded nonwoven fabric, a melt blown nonwoven fabric, and a nonwoven fabric in which one or more polypropylene ultrafine fiber melt blown nonwoven fabric layers are sandwiched between spunbonded nonwoven fabric layers, such as an SMS nonwoven fabric or an SMMS nonwoven fabric, can be suitably used. A nonwoven fabric used for the reinforcing layer 53 preferably has a fineness of about 0.5 to 2.5 dtex, and preferably has a basis weight of about 10 to 30 g/m². A nonwoven fabric used for the reinforcing layer 53 preferably has a thickness of about 0.1 to 10 mm.

As a filament assembly used for the reinforcing layer 53, one obtained by opening a tow (fiber bundle) of synthetic fibers such as cellulose acetate as necessary can be suitably used. The filament assembly can have a fiber basis weight of about 30 to 120 g/m², for example. The filament has a fineness of, for example, 1 to 16 dtex, preferably 1 to 10 dtex, more preferably 1 to 5 dtex. The filament may be a non-crimped fiber but is preferably a crimped fiber. The degree of crimp of the crimped fibers can be, for example, about 5 to 75, preferably 10 to 50, and more preferably 15 to 50 per inch. A uniformly crimped fiber is preferably used. The use of the crimped fiber makes it possible to manufacture a bulky and lightweight absorber, and makes it possible to easily manufacture a highly integrated filament assembly due to entanglement of fibers. The cross-sectional shape of the filament is not particularly limited, and may be, for example, circular, elliptical, irregular (for example, Y-shaped, X-shaped, I-shaped, or R-shaped) , or hollow. As the filament assembly, for example, a bundle of 3,000 to 1,000,000, preferably about 5,000 to 1,000,000 filaments can be used.

A width 53w of the reinforcing layer 53 can be determined appropriately, and as illustrated in Fig. 12 (c), can be a width projecting from both side edges of the widest portions of the upper accumulation layer 51 and the lower accumulation layer 52. However, as illustrated in Figs. 12(a) and 12(b), it is desirable that the width 53w has a width extending in a width direction range the same as or narrower than a width direction range of the widest portions of the upper accumulation layer 51 and the lower accumulation layer 52. In particular, as illustrated in Fig. 12 (a), in a case where the narrower portion 54 is disposed in an intermediate portion of the upper accumulation layer 51 and the lower accumulation layer 52 in a front-back direction to improve fitting with respect to a periphery of a leg, disposition of the reinforcing layer 53 in a width direction range of the narrowest portion of the narrower portion 54 is preferable because flexibility of a portion located in the narrower portion 54 is not reduced by the reinforcing layer 53.

The length of the reinforcing layer 53 in a front-back direction only needs to be determined appropriately. However, in a case of the absorbent element 50 wrapped with the wrapping sheet 58, when the reinforcing layer 53 is cut at cutting positions on the front and back ends, manufacture is easy. Therefore, it is desirable that the length of the reinforcing layer 53 in a front-back direction is equal to the length of the absorbent element 50 in the front-back direction. However, although not illustrated, the length of the reinforcing layer 53 in the front-back direction can be equal to a longer one of the lengths of the upper accumulation layer 51 and the lower accumulation layer 52 in the front-back direction, or can be shorter than a shorter one of the lengths of the upper accumulation layer 51 and the lower accumulation layer 52 in the front-back direction.

### (Wrapping sheet)

As the wrapping sheet 58, a liquid pervious material such as tissue paper, particularly, crepe paper, a nonwoven fabric, a polylaminated nonwoven fabric, or a sheet with small holes can be used. However, it is desirable that the wrapping sheet 58 is a sheet from which super absorbent polymer particles do not escape. In a case where a nonwoven fabric is used instead of crepe paper, a hydrophilic SMS nonwoven fabric (SMS, SSMMS, or the like) is particularly suitable, and polypropylene, a polyethylene/polypropylene composite material, or the like can be used as a material thereof. A nonwoven fabric having a basis weight of 5 to 40 g/m², particularly of 10 to 30 g/m² is desirable.

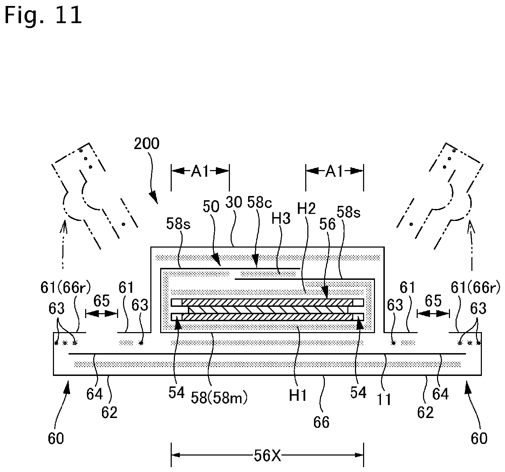

As illustrated in Figs. 3 and 11, as a wrapping mode of the wrapping sheet 58, the wrapping sheet 58 is wound around the absorber 56 cylindrically so as to surround front and back surfaces and both side surfaces of the absorber 56 from viewpoints of ease of manufacture and prevention of leakage of super absorbent polymer particles from front and back edges. More specifically, the wrapping sheet 58 has an intermediate portion 58m located on one side of the front and back of the absorber 56, both side portions 58s folded back from the intermediate portion 58m to the other side of the absorber 56, and a connecting portion 58c is formed sheet 58 on the other side of the absorber 56. The front and back end portions of the wrapping sheet 58 project fro by overlapping tip portions of both side portions 58s of the wrapping m the front and back of the absorber 56, and are directly bonded to each other without the absorber 56 interposed therebetween at the projecting portion.

The connecting portion 58c of the wrapping sheet 58 may be located on the front surface side or the back surface side of the absorber 56. However, in a mode in which a weight ratio of fibers : super absorbent polymer particles is 50 : 50 to 20 : 80 in the absorber 56 (that is, the absorber 56 has a high content of super absorbent polymer particles), and the content of super absorbent polymer particles in the absorber 56 stepwise or continuously increases from the back surface side to the front surface side, the connecting portion 58c is preferably located on the back surface side of the absorber 56, that is, on the opposite side to a portion in which the content of super absorbent polymer particles is high in the absorber 56. As a result, even if bonding of the connecting portion 58c of the wrapping sheet 58 is insufficient, a gap is not easily generated in the connecting portion 58c. In addition, even if super absorbent polymer particles released from the absorber 56 leak from the connecting portion 58c of the wrapping sheet 58, the connecting portion 58c is located on the back surface side of the absorbent element 50, and therefore there is a little risk that the super absorbent polymer particles leak to a skin side of a wearer. Furthermore, an overlapping portion of the wrapping sheet 58 has high liquid retention compared with the other portions, and therefore may promote returning if the overlapping portion is located on the front surface side of the absorber 56. However, if the overlapping portion is located on the back surface side of the absorber 56, such a problem does not occur.

### (Bonding structure of wrapping sheet and absorber)

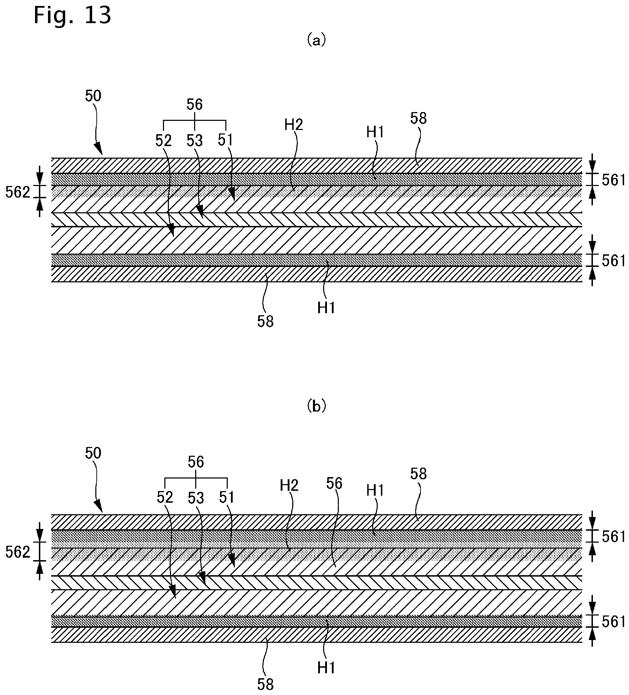

In an inner surface of the wrapping sheet 58, it is desirable that the entire portion facing the absorber 56 is bonded to an outer surface of the absorber 56 through hot melt adhesives H1 and H2. In addition, it is desirable that overlapping portions of end portions of the wrapping sheet 58 in the connecting portion 58c are bonded to each other through a hot melt adhesive H3. It is desirable that the hot melt adhesives H1 and H2 for bonding the wrapping sheet 58 and the absorber 56 permeate the absorber 56 to some extent to form an impregnation layer 562 as illustrated in Fig. 13, and the hot melt adhesives H1 and H2 form an adhesive layer 561 by hardly permeating the wrapping sheet 58 or by permeating the wrapping sheet 58 less than permeating the absorber 56.

The use amount of the hot melt adhesives H1 and H2 for bonding the wrapping sheet 58 and the absorber 56 is not particularly limited. However, at least the amount in a region A1 at each of both end portions in a width direction on a side having the connecting portion 58c of the wrapping sheet 58 in the absorber 56 is preferably larger than the amount on the opposite side of the absorber 56. As a result, bonding strength of the wrapping sheet 58 and the absorber 56 on a side where bonding strength is easily reduced (a side having the connecting portion 58c) can be enhanced, the total use amount of the hot melt adhesives H1 and H2 for bonding the absorber 56 and the wrapping sheet 58 can be suppressed, and shape collapse of the absorber 56 can be effectively suppressed. That is, in the wrapping sheet winding type absorbent element 50, at the time of manufacture, when the absorber 56 is transferred, a lower surface of the absorber 56 is bonded to an intermediate portion of the wrapping sheet 58, and then both side portions of the wrapping sheet 58 are folded back and bonded to an upper surface of the absorber 56. Therefore, both the front surface side and the back surface side of the absorber 56 cannot be bonded under the same conditions. Here, when both side portions of the wrapping sheet 58 are folded back and bonded to an upper surface of the absorber 56, by previously applying the hot melt adhesives H1 and H2 to the upper surface of the absorber 56 and folding back and bonding the both side portions of the wrapping sheet 58 thereto, the shape of the upper surface of the absorber 56 is stabilized advantageously. Therefore, such a method has been adopted. However, a surface of the absorber 56 has a low density and large unevenness even when being pressed by a press apparatus . Therefore, when the hot melt adhesives H1 and H2 are applied to the absorber 56 and bonded to the wrapping sheet 58, bonding is relatively weak as compared with a case where the hot melt adhesives H1 and H2 are applied to the wrapping sheet 58 and bonded to the absorber 56 with the same use amount (weight per unit area, the same applies hereinafter) of the hot melt adhesives H1 and H2. Therefore, it is desirable to enhance bonding strength of the wrapping sheet 58 and the absorber 56 on a side having the connecting portion 58c as described above.

The use amount of the hot melt adhesives H1 and H2 for bonding the wrapping sheet 58 and the absorber 56 can be determined appropriately, but is preferably 12 to 25 g/m² in the large amount region A1 and 9 to 15 g/m² in a small amount region (other than A1) . The use amount in the large amount region A1 is preferably about 1.1 to 1.4 times the use amount in the small amount region.

As described above, when a weight ratio of fibers : super absorbent polymer particles is 50 : 50 to 20 : 80 in the absorber 56 (that is, the absorber 56 has a high content of super absorbent polymer particles), shape collapse of the absorber 56 at both end portions in a width direction easily occurs. Therefore, reinforcement with the large amount region A1 of the hot melt adhesives H1 and H2 is preferably performed.

In addition, as in the illustrated embodiments, in a case where the narrower portion 54 for improving fitting with respect to a periphery of a legis formed in the absorber 56, shape collapse of the narrower portion 54 easily occurs. Therefore, a form is preferable in which the large amount region A1 of the hot melt adhesives H1 and H2 is set as a region including the entire narrower portion 54 in a width direction to suppress shape collapse of the absorber 56 in the narrower portion 54.

As illustrated in Fig. 12(c), the use amount of the hot melt adhesives H1 and H2 maybe changed by changing the application amount in one application depending on a site. However, this is often difficult. Therefore, as in embodiments illustrated in Figs. 3, 7, 11, 12(a), and 12(b), the use amount of the hot melt adhesives H1 and H2 is preferably changed by changing the number of the coated layers (that is, the number of coats) of the hot melt adhesives H1 and H2 depending on a site.

In a case where the use amount of the hot melt adhesives H1 and H2 is changed depending on the number of the coated layers of the hot melt adhesives H1 and H2 as described above, the embodiment is also preferable in which at least one of a coating pattern and the type of hot melt adhesive is changed depending on an application layer. As a result, it is possible to change the coating pattern and the types of hot melt adhesives H1 and H2 depending on an application target in a simple manufacturing process and to locally increase the use amount of the hot melt adhesives H1 and H2.

In particular, as illustrated in Fig. 13, the embodiment is preferable in which the absorber 56 has the impregnation layer 562 impregnated with the hot melt adhesive H2 having a melt viscosity of 1000 to 6000 mPa·s (preferably 2000 to 5500 mPa·s) on a side having the connecting portion 58c, a portion facing the impregnation layer 562 on an inner surface of the wrapping sheet 58 is bonded to an outer surface of the impregnation layer 562 through the adhesive layer 561 formed of the hot melt adhesive H1 having a melt viscosity of 4000 to 9000 mPa.s (preferably 6000 to 8000 mPa·s), and the hot melt adhesive H2 with which the impregnation layer 562 is impregnated has a lower viscosity than the hot melt adhesive H1 forming the adhesive layer 561. A portion of the absorber 56 not having the impregnation layer 562 can be bonded to the inner surface of the wrapping sheet 58 only through the adhesive layer 561. As described above, with a structure in which the hot melt adhesives H1 and H2 having different melt viscosities are suitably used at suitable sites, at the time of forming the impregnation layer 562, the absorber 56 can be sufficiently impregnated with the hot melt adhesive H2 because the hot melt adhesive H2 has a sufficiently low melt viscosity, thus enabling to effectively stabilize the shape of the absorber 56. In addition, at the time of forming the adhesive layer 561, the hot melt adhesive H1 is unlikely to permeate the absorber 56 and the wrapping sheet 58 because the hot melt adhesive H1 has a high melt viscosity, an adhesive property of the absorber 56 and the wrapping sheet 58 is good, and therefore shape collapse of the absorber 56 can be suppressed.

As illustrated in Fig. 13, the absorber 56 is impregnated with the hot melt adhesive H2, and the impregnation layer 562 is formed in a range from an impregnation surface to a certain depth. The thickness of the impregnation layer 562 is not particularly limited, but is preferably about 10 to 50% of the thickness 56t of the absorber 56. The entire hot melt adhesive H2 of the impregnation layer 562 in a thickness direction may permeate the absorber 56 as illustrated in Fig. 13(a), or a part thereof in the thickness direction may remain on the absorber 56 as illustrated in Fig. 13(b).

As illustrated in Fig. 13, the adhesive layer 561 is a layer of the hot melt adhesive H1 mainly located between the wrapping sheet 58 and the impregnation layer 562 of the absorber 56, and the wrapping sheet 58 is impregnated with the hot melt adhesive H1 to some extent. The absorber 56 is hardly impregnated with the hot melt adhesive H1 in a portion having the impregnation layer 562, and is impregnated therewith in a portion not having the impregnation layer 562 more than in the portion having the impregnation layer 562.

The composition of each of the hot melt adhesive H2 of the impregnation layer 562 and the hot melt adhesive H1 of the adhesive layer 561 is not particularly limited as long as having a melt viscosity in the above range, but a rubber-based hot melt adhesive is preferably used from viewpoints of softness and odor. In addition, the hot melt adhesive preferably has a functional group in order to increase polarity. By increasing the polarity of the hot melt adhesive, bonding to the pulp/ super absorbent polymer/wrapping sheet 58 can be strengthened when being wet due to an intermolecular force. Furthermore, as the hot melt adhesive H2 of the impregnation layer 562, a delayed crystalline type adhesive is preferably used. The delayed crystalline type adhesive means a hot melt adhesive which is not crystallized immediately after application and is solidified after permeating between fibers. Even if a large amount of the adhesive is applied, the adhesive does not easily exude, and sufficiently permeates the absorber 56 side with time, and bonding strength between the wrapping sheet 58 and the absorber 56 can be enhanced.

### (Method for manufacturing absorbent element)

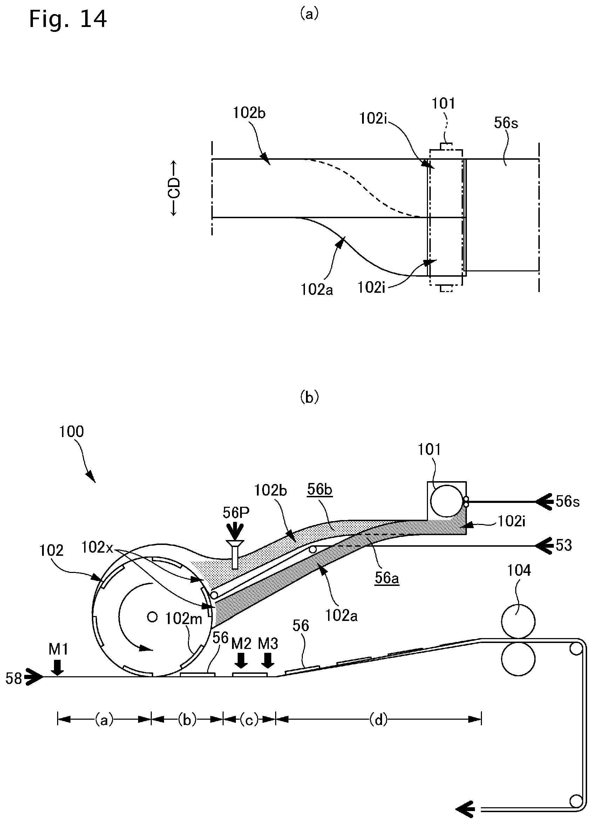

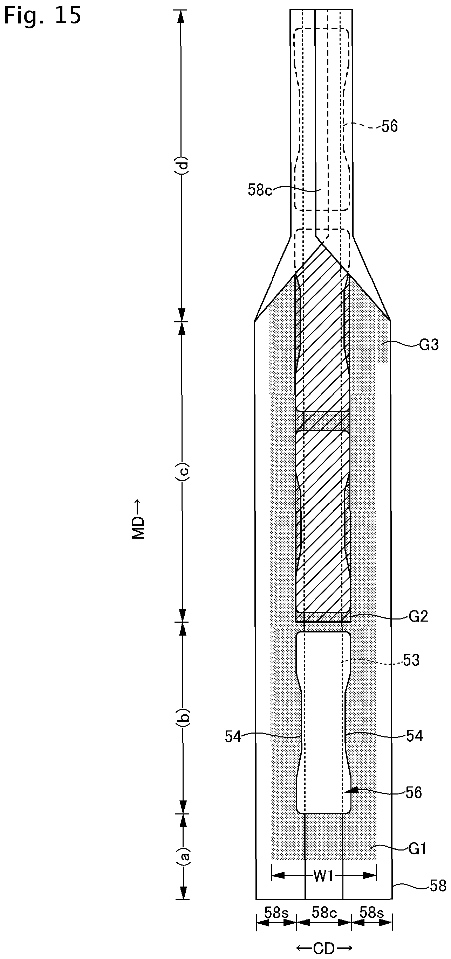

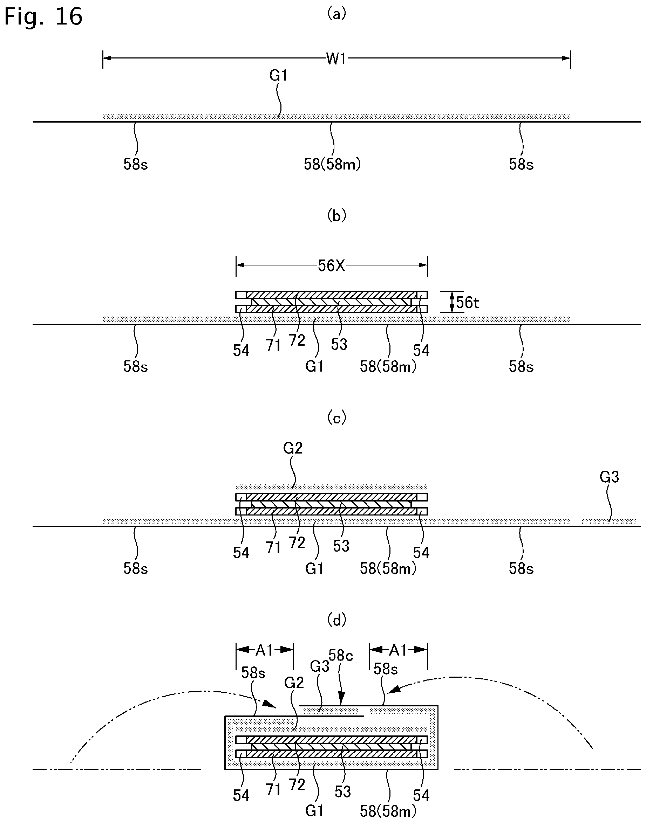

Figs. 14 to 16 illustrate examples of equipment and steps for manufacturing the above-described absorbent element 50. Sections (a) to (d) illustrated in Figs. 14 and 15 correspond to the cross-sectional states of (a) to (d) illustrated in Fig. 16. In the manufacturing equipment 100, the short fiber sheet 56s such as a continuous belt-shaped pulp sheet is continuously supplied to the defibrating machine 101 in a longitudinal direction, and a tip portion thereof is sequentially defibrated. Defibrated products 56a and 56b obtained by this fibrillation as an accumulation material are scattered and supplied from obliquely upward to an outer peripheral surface of the laterally disposed fiber accumulating drum 102 to be rotationally driven by an electric motor (not illustrated) or the like through a first chamber 102a and a second chamber 102b, respectively. Outlets 102x of the first chamber 102a and the second chamber 102b are opened toward a first accumulation position and a second accumulation position (range of a rotational direction of the fiber accumulating drum) disposed in this order in a rotational direction of the fiber accumulating drum 102. In the illustrated embodiment, the super absorbent polymer particles 56P are supplied from a supply port disposed in the middle of the second chamber 102b, and the defibrated product 102b mixed with the super absorbent polymer particles 56P is supplied to the fiber accumulating drum 102. However, alternatively or additionally, the super absorbent polymer particles 56P may be supplied from a supply port disposed in the middle of the first chamber as necessary, and the defibrated product 102a mixed with the super absorbent polymer particles 56P may be supplied to the fiber accumulating drum 102. On an outer peripheral surface of the fiber accumulating drum 102, the absorber mold 102m, the front-back direction of which is a rotational direction of the accumulating drum 102, is formed in a recessed shape, and many suction holes (not illustrated) are formed on a bottom surface of the absorber mold 102m. At the first accumulation position and the second accumulation position corresponding to the outlets 102x of the first chamber 102a and the second chamber 102b, the accumulation material supplied from the chambers 102a and 102b is sequentially accumulated in the absorber mold 102m by suction from the suction holes in the absorber mold 102m.

Characteristically, the first accumulation position and the second accumulation position, that is, the outlets 102x of the first chamber 102a and the second chamber 102b are disposed at intervals in a rotational direction of the fiber accumulating drum 102, and the continuous belt-shaped air-permeable reinforcing layer 53 is supplied in the rotational direction of the fiber accumulating drum 102 between the first accumulation position and the second accumulation position and passing over the absorber mold 102m on the outer peripheral surface of the fiber accumulating drum 102. As a result, in the absorber mold 102m, first, the accumulation material supplied from the first chamber 102a is accumulated by suction to form a first accumulation layer 71 at the first accumulation position, and then the reinforcing layer 53 is stuck to a top surface of the first accumulation layer 71 by suction. Thereafter, the accumulation material supplied from the second chamber 102b is accumulated by suction to form a second accumulation layer 72 at the second accumulation position. In this way, it is possible to manufacture the absorber 56 in which the reinforcing layer 53 is sandwiched between the plurality of accumulation layers 71 and 72 with the single fiber accumulating drum 102 as illustrated in Fig. 16. Therefore, the manufacturing equipment 100 is simple and compact, and equipment cost and power consumption cost are not high.

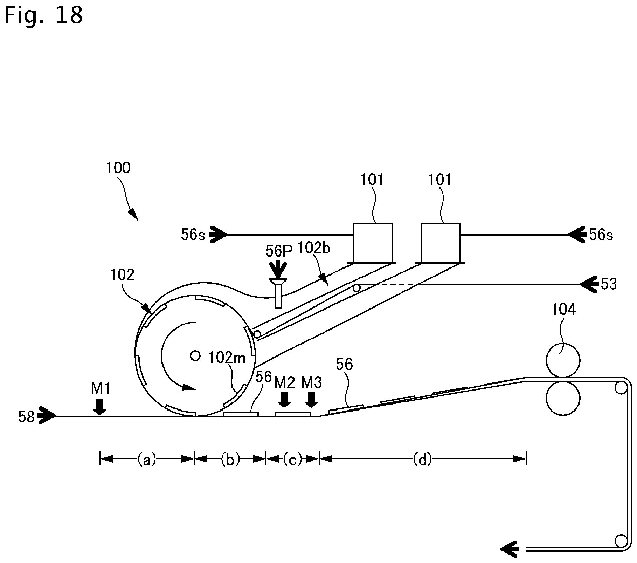

The defibrating machine 101 is not particularly limited as long as being able to perform continuous processing. The defibrating machine 101 in the illustrated embodiment has a roll-shaped crushing blade to be rotationally driven with the CD as an axial center by an electric motor or the like, and a tip portion of the short fiber sheet 56s continuously supplied to an outer peripheral surface of the crushing blade is brought into contact therewith, and defibration is sequentially performed. In the defibrating machine 101, the defibrated product of the short fiber sheet is dropped and discharged downward from a defibrating position, and inlets 102i of the first chamber 102a and the second chamber 102b are disposed below the defibrating position. Therefore, the thicknesses (use amounts of fibers) of the accumulation layers 71 and 72 can be changed depending on the sizes of the inlets 102i of the first chamber 102a and the second chamber 102b in the CD, and the thickness of one of the accumulation layers can be larger than or the same as that of the other accumulation layer. By transporting the defibrated product defibrated by the single defibrating machine 101 to the fiber accumulating drum 102 through an independent route after the defibration, an apparatus can be further miniaturized. Of course, as illustrated in Fig. 18, the defibrating machine 101 can be disposed for each chamber to make the defibration and transportation to the fiber accumulating drum 102 completely independent.

Disposition of the inlets 102i of the first chamber 102a and the second chamber 102b can be appropriately determined depending on the type of the defibrating machine 101 and a direction of discharge of a defibrated product, and can be disposed in the MD. However, in a case of such an ordinary defibrating machine 101 as in the illustrated embodiment, the CD in the defibrating machine 101 is preferably parallel to a rotation axis of the fiber accumulating drum 102. Therefore, as illustrated in Fig. 14, the inlets 102i of the first chamber 102a and the second chamber 102b are preferably disposed in the CD corresponding to defibrating positions of the short fiber sheet 56s in the defibrating machine 101 in the CD. Meanwhile, the outlets 102x of the first chamber 102a and the second chamber 102b are disposed so as to face an outer peripheral surface of the fiber accumulating drum 102 in a circumferential direction of the fiber accumulating drum 102. That is, the supply chambers 102a and 102b are disposed in the CD on the inlet 102i side, and form passages gradually curved in the CD up to a position facing an outer peripheral surface of the fiber accumulating drum 102 as necessary while the positions thereof in an up-down direction are shifted toward the outlet 102x side. By disposing the supply chambers 102a and 102b three-dimensionally in this way, it is possible to supply a defibrated product to each of the supply chambers 102a and 102b without changing a positional relationship of the defibrated product in the defibratingmachine 101. In this case, an entrance of a reinforcing layer is disposed between the outlets 102x of the first chamber 102a and the second chamber 102b disposed in a circumferential direction of the fiber accumulating drum 102.

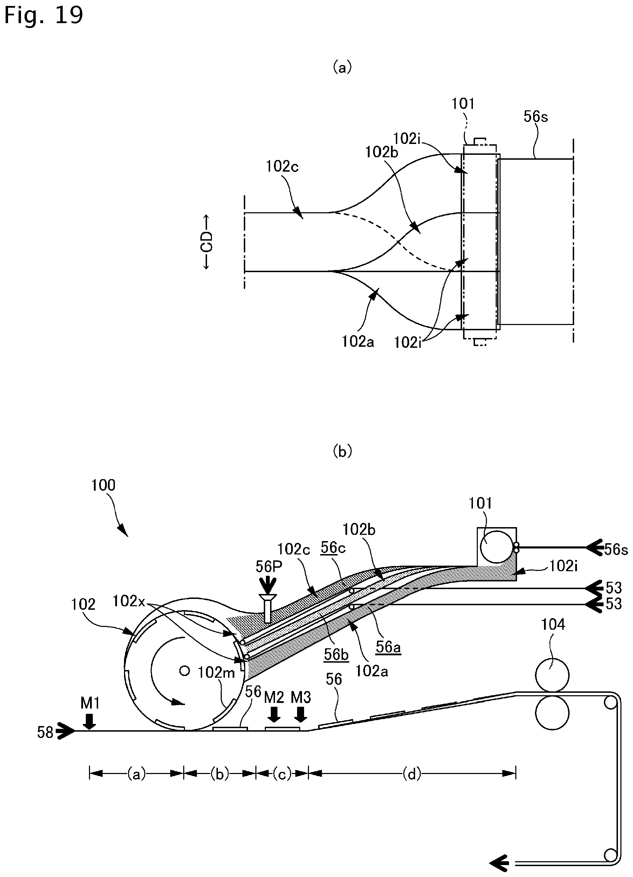

The number of supply chambers 102a and 102b only needs to be equal to or larger than the number of accumulation layers in the absorber 56. The embodiment illustrated in Fig. 14 assumes a case where the above-described absorber 56 having a three-layered structure is formed. As in the embodiment illustrated in Fig. 19, the number of layers can be increased by further increasing the number of supply chambers, or alternatively or additionally by increasing the number of reinforcing layers. In the embodiment illustrated in Fig. 19, the first chamber 102a, the second chamber 102b, and a third chamber are disposed, and accumulation materials 56a, 56b, and 56c are supplied from the first chamber 102a, the second chamber 102b, and the third chamber at a first accumulation position, a second accumulation position, and a third accumulation position disposed in this order in a rotational direction of the fiber accumulating drum 102, respectively. In addition, the reinforcing layer 53 is supplied separately between the outlets 102x of the first chamber 102a and the second chamber 102b, and between the outlets 102x of the second chamber 102b and the third chamber 102c to form an absorber having five layers in total.

In a case where a plurality of supply chambers for the fiber accumulating drum 102 is disposed in a drum rotational direction as in this example, the amount of the super absorbent polymer 56P supplied to the supply chamber 102a can be different from that supplied to the supply chamber 102b. The position of each layer in the absorber 56 is determined by the positions of the outlets 102x of the supply chambers 102a and 102b in a circumferential direction of the fiber accumulating drum 102. When the position of the outlet 102x is located on the opposite side in a rotational direction of the fiber accumulating drum 102, fibers are supplied first into the absorber mold 102m, and are accumulated on a bottom side (suction hole side) in the absorber mold 102m. Therefore, by appropriately adjusting the amounts of the super-absorbent polymer 56P supplied to the supply chambers 102a and 102b, a distribution of the super absorbent polymer particles of a product in a thickness direction of the absorber 56 can be selected. For example, in the case illustrated in Fig. 14, by supplying the super absorbent polymer 56P only to the supply chamber 102b on a rotational direction side, a layer hardly containing the super absorbent polymer particles 56P is formed on the bottom side (suction hole side) in the absorber mold 102m, and a layer containing the super absorbent polymer particles 56P is formed on an inlet side of the absorber mold 102m. However, the super absorbent polymer 56P can also be supplied to the opposite supply chamber 102a. In this case, the layered structure in the absorber mold 102m is also reversed. However, in the latter case, since many super absorbent polymer particles 56P are contained on the suction hole side of the absorber mold 102m in the fiber accumulating drum 102, the suction holes of the absorber mold 102m are easily clogged with the super absorbent polymer particles 56P. Meanwhile, in the former case, the first accumulation layer 71 and the reinforcing layer 53 act as a barrier, it is difficult for the super absorbent polymer particles 56P to reach the suction holes of the absorber mold 102m, and the suction holes of the absorber mold 102m are less likely to be clogged with the super absorbent polymer particles 56P advantageously. In addition, as described later, for example, also by determining the positions of the front and back (keeping the state or reversing the state) when the manufactured absorbent element 50 is assembled to another member, a distribution of the super absorbent polymer particles of a product in a thickness direction of the absorber 56 can be selected.

When the reinforcing layer 53 is formed of a nonwoven fabric, the super absorbent polymer particles 56P try to move from the second accumulation layer 72 to the first accumulation layer 71 by suction. At this time, some of the particles pass through the reinforcing layer 53 formed of a nonwoven fabric to reach the first accumulation layer 71. However, many of the particles are captured by the reinforcing layer 53 formed of a nonwoven fabric, and the amount of polymer in a region in which the reinforcing layer 53 is substantially centrally located in a thickness direction is the largest. A layer with a large amount of polymer tends to reduce strength. However, the substantially centrally located reinforcing layer 53 formed of a nonwoven fabric functions as a core material, and therefore reduction in strength is small even in the absorber 56 with a large amount of polymer. Furthermore, a nonwoven fabric is an assembly of fibers with gaps. Therefore, when the reinforcing layer 53 is a nonwoven fabric layer, the first accumulation layer 71 and the second accumulation layer 72 easily adhere thereto. In particular, the second accumulation layer 72 is accumulated on the reinforcing layer 53, and is therefore highly integrated with the reinforcing layer 53.

When the absorber 56 formed in the absorber mold 102m is located so as to face the continuous belt-shaped wrapping sheet 58 supplied along the outer peripheral surface of the fiber accumulating drum 102 by rotation of the fiber accumulating drum 102, the absorber 56 is released from the mold and transferred onto the wrapping sheet 58. On the transfer surface of the wrapping sheet 58, to which the absorber 56 is transferred, a first adhesive layer G1 is formed in advance by applying a hot melt adhesive M1, and the absorber 56 is bonded to the wrapping sheet 58 with the first adhesive layer G1. By sequentially performing the first bonding step, the absorber 56 is intermittently supplied onto the wrapping sheet 58 continuously transferred, and bonding is sequentially performed.

In the illustrated embodiment, the width of the wrapping sheet 58 in the CD is wider than that of the absorber 56. After the absorber 56 is transferred onto the intermediate portion 58m of the wrapping sheet 58 in the CD, the hot melt adhesive M2 is applied over the maximum width of an upper surface of the absorber 56 to form the second adhesive layer G2. The second adhesive layer G2 is an adhesive layer mainly intended to enhance shape maintainability of the absorber 56 by impregnation into the absorber 56, but also functions as bonding to the wrapping sheet 58. In addition, by applying a hot melt adhesive M3 to one end portion of the wrapping sheet in the CD as necessary, a third adhesive layer G3 for bonding a connecting portion is formed.

Incidentally, prior to application of the hot melt adhesive M2 for forming the second adhesive layer G2, when accumulation is performed such that many super absorbent polymer particles 56P are contained in a bottom side of the absorber mold 102m in the fiber accumulating drum 102, and the product thus obtained is transferred onto the wrapping sheet 58 (at this time, the upper and lower sides of the absorber 56 are replaced with each other), many super absorbent polymer particles 56P are contained in an upper surface side of the absorber 56. Therefore, when the hot melt adhesive M2 is applied to the upper surface of the absorber 56, the super absorbent polymer particles 56P easily scatter due to the application force disadvantageously. Meanwhile, when accumulation is performed in such a manner that the content of the super absorbent polymer particles increases stepwise or continuously from the bottom side to the inlet side of the absorber mold 102m in the fiber accumulating drum 102, in application of the hot melt adhesive M2 to the upper surface of the absorber 56, the content of the super absorbent polymer particles 56P becomes lower toward an upper surface side of the absorber 56, and the super absorbent polymer particles are less likely to scatter.

After application of the second adhesive layer G2, both side portions 58s projecting from both sides of the absorber 56 in the wrapping sheet 58 in the CD are folded back at positions along both side edges of the absorber 56 by a sailor (not illustrated), and bonded to an upper surface of the absorber 56 (second bonding step). Furthermore, both end portions in the CD overlap with each other, and bonded with the third adhesive layer G3 formed by applying the hot melt adhesive M3 in advance to the overlapping portion to form the connecting portion 58c. For such bonding, after the wrapping sheet 58 is folded back, the wrapping sheet 58 can be press-bonded through a pair of press bonding rolls 104. In this way, a continuous body of the absorbent element 50 in which the absorber 56 is intermittently fixed in the MD is formed in the cylindrical continuous body of the wrapping sheet 58 continuous in the MD.

Incidentally, in a case where an underpants-type disposable diaper as in the present embodiment is manufactured, a continuous body of the absorbent element 50 manufactured through the first bonding step and the second bonding step is sandwiched between the continuous belt-shaped top sheet 30 and the continuous belt-shaped liquid impervious sheet 11, and a continuous body of the around-leg gather 60 is further attached thereto as necessary. Thereafter, the resulting product is intermittently cut in the MD into individual inner bodies 200, then the inner bodies 200 are attached to a continuous body of the outer bodies 12F and 12B manufactured separately, and folded such that the front and back overlap with each other to form the side seal portion 12A, and the resulting product is cut into individual diapers . In a case where a pad-type disposable diaper or a tape-type disposable diaper is manufactured, a continuous body of an absorbent element is sandwiched between a continuous belt-shaped top sheet and a continuous belt-shaped liquid impervious sheet, and a continuous body of around-leg gather is further attached thereto as necessary (in a case of the tape-type disposable diaper, a fastening tape is also attached) . Thereafter, the resulting product is intermittently cut in the MD into individual diapers.

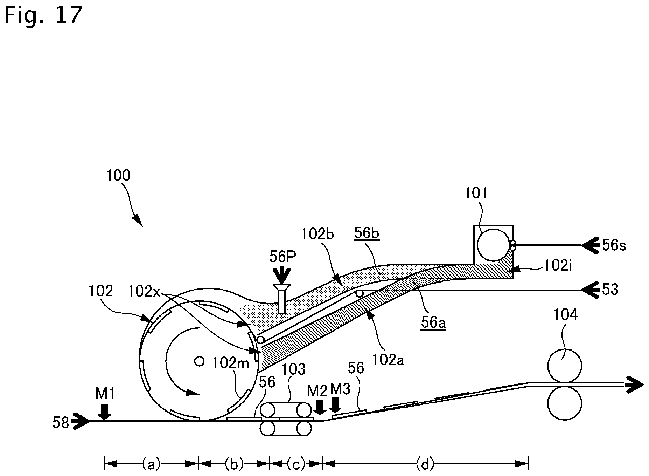

As described above, in a case where the connecting portion 58c of the wrapping sheet 58 is located on a back surface side of the absorber 56, as in the illustrated embodiment, after the absorbent element 50 is manufactured with a direction immediately behind the fiber accumulating drum 102, it is only required to reverse the absorbent element 50 upside down by reversing the absorbent element 50 with a roll, to attach a member to be mounted on a front surface side of the absorber 56 in the absorbent article to the upper side, and to attach a member to be mounted on a back surface side of the absorber 56 in the absorbent article to the lower side. In this case, the first accumulation layer 71 serves as the lower accumulation layer 52 described above, and the second accumulation layer 72 serves as the upper accumulation layer 51 described above. Without reversing the the absorbent element 50 upside down, a member to be mounted on a back surface side of the absorber 56 in the absorbent article can be attached to the upper side, and a member to be mounted on a front surface side of the absorber 56 in the absorbent article can be attached to the lower side. In a case where the connecting portion 58c of the wrapping sheet 58 is located on a front surface side of the absorber 56, as illustrated in Fig. 17, without reversing the direction of the absorbent element 50 upside down with the direction immediately behind the fiber accumulating drum 102, it is only required to attach a member to be mounted on a front surface side of the absorber 56 in the absorbent article to the upper side, and to attach a member to be mounted on a back surface side of the absorber 56 in the absorbent article to the lower side. In this case, the first accumulation layer 71 serves as the upper accumulation layer 51 described above, and the second accumulation layer 72 serves as the lower accumulation layer 52 described above.

An application width W1 of the first adhesive layer G1 and an application width of the second adhesive layer G2 can be determined appropriately. However, as in the first example illustrated in Figs. 14 to 16, preferably, in the first bonding step (a) and (b), the first adhesive layer G1 is formed on the wrapping sheet 58 so as to be wider than the sum of the width 56X of the absorber 56 and the thickness 56t of the absorber 56, and in the second bonding step (c) and (d), at least both end portions of the absorber 56 in a width direction are bonded to a folded portion and an upper surface of the absorber 56 through the first adhesive layer G1 applied to the folded portion and the second adhesive layer G2 applied to the upper surface of the absorber 56. As a result, on a lower surface side of the absorber 56 (the opposite side to the side having the connecting portion 58c in the wrapping sheet 58), an inner surface of the wrapping sheet 58 is bonded to the absorber 56 only with the first adhesive. Meanwhile, on an upper surface side of the absorber 56 (the side having the connecting portion 58c in the wrapping sheet 58), at least in a region of both end portions in a width direction, the inner surface of the wrapping sheet 58 is bonded to the absorber 56 with the two layers of the first adhesive layer G1 and the second adhesive layer G2. As a result, on an upper surface side of the absorber 56 (side having the connecting portion 58c in the wrapping sheet 58) in which bonding strength is more easily reduced than a lower surface side of the absorber 56, the use amount of the hot melt adhesives H1 and H2 at least in a region of both end portions in a width direction can be larger than that on the opposite side of the absorber 56. A side surface of the absorber 56 can also be bonded without a gap. Therefore, it is possible to effectively suppress shape collapse of the absorber 56 while suppressing the total use amount of the hot melt adhesives H1 and H2 for bonding the absorber 56 and the wrapping sheet 58.

Incidentally, as described above, in a case where the first adhesive layer G1 is formed so as to be wider than the sum of the width of the absorber 56 and the thickness of the absorber 56, as illustrated in Fig. 17, when a pressing step is performed over the maximum width of the absorber 56 with a press apparatus 103 from the first bonding step (a) and (b) to the second bonding step (c) and (d), the first adhesive layer G1 projecting from both sides of the absorber 56 in a width direction may be attached to the press apparatus 103. Therefore, from the first bonding step (a) and (b) to the second bonding step (c) and (d), it is desirable that a pressing step of pressing the absorber 56 over the maximum width is not performed. Alternatively, in a case where the pressing step is performed, it is desirable that pressing is performed with a width narrower than the maximum width of the absorber 56 or the application width W1 of the first adhesive layer is made narrower than the width 56X of the absorber 56.

The application width W1 of the first adhesive layer G1 only needs to be wider than the sum of the width 56X of the absorber 56 and the thickness 56t of the absorber 56. However, as in the illustrated embodiment, in a case where the absorber 56 has the narrower portion 54 along a periphery of a leg, the application width W1 is preferably wider than a width obtained by further adding a recess width W2 of the narrower portion 54 to both sides in the CD. As a result, a portion bonded with the two layers of the first adhesive layer G1 and the second adhesive layer G2 reaches an edge of the narrower portion 54, and the wrapping sheets 58 are strongly bonded to each other at a recessed site of the narrower portion 54 with the three layers of the first adhesive layer G1 on the connecting portion 58c side, the second adhesive layer G2, and the first adhesive layer G1 on the opposite side. Therefore, shape collapse of the absorber 56 in the narrower portion 54 can be effectively prevented.

Furthermore, as in the embodiment illustrated in Fig. 12 (b), the application width W1 of the first adhesive layer G1 may be extended to the overlapping portion of the wrapping sheets 58, and the overlapping portion may be bonded with the first adhesive layer G1.

A coating pattern of the hot melt adhesives M1 and M2 for forming the first adhesive layer G1 and the second adhesive layer G2 is not particularly limited. However, the first adhesive layer G1 is applied to a wide range of the wrapping sheet 58 and is a basis of bonding between the wrapping sheet 58 and the absorber 56 (one forming the above-described adhesive layer 561) . Therefore, permeability into the wrapping sheet 58 and the absorber 56 does not necessarily have to be high. Meanwhile, the second adhesive layer G2 is mainly intended to enhance shape maintainability of the absorber 56 by impregnation into the absorber 56 (one forming the above-described impregnation layer 562). Therefore, permeability into the absorber 56 is preferably high. The hot melt adhesive M1 constituting the first adhesive layer G1 is preferably applied in a spiral shape or a mesh shape, and the hot melt adhesive M2 constituting the second adhesive layer G2 is preferably applied in a continuous surface shape from such a viewpoint.

For the same reason, the hot melt adhesive M1 for forming the first adhesive layer G1 preferably has a melt viscosity of 4000 to 9000 mPa·s, and the hot melt adhesive M2 for forming the second adhesive layer G2 preferably has a melt viscosity of 1000 to 6000 mPa·s. The viscosity of the hot melt adhesive M2 for forming the second adhesive layer G2 is preferably lower than that of the hot melt adhesive M1 for forming the first adhesive layer G1.

### (Around-Leg gather)

The around-leg gather 60 extends along both sides of an absorption surface of the inner body 200 in a width direction and rises toward a periphery of a wearer's leg, and is disposed in order to block urine and loose stool moving laterally along the top sheet 30 to prevent side leakage.

As illustrated in Figs. 3 and 4, the around-leg gather 60 of the present embodiment includes an inner nonwoven fabric layer 61 constituting an inner surface in a width direction, an outer nonwoven fabric layer 62 constituting an outer surface in the width direction, an elastically stretchable gather member 63 disposed in a front-back direction between the inner nonwoven fabric layer 61 and the outer nonwoven fabric layer 62 at least at a tip portion in an intermediate portion in the front-back direction, and a liquid impervious sheet 64 (11) extending over a range from a base to a position closer to a tip side than the base and sandwiched between the inner nonwoven fabric layer 61 and the outer nonwoven fabric layer 62. In the illustrated embodiment, a portion having the liquid impervious sheet 64 and closer to the base side than the tip portion in the around-leg gather 60 serves as a nonwoven fabric absent portion 65 in which the inner nonwoven fabric layer 61 is absent and the liquid impervious sheet 64 is exposed over the entire around-leg gather 60 in a front-back direction. In this way, by disposing the nonwoven fabric absent portion 65 without the inner nonwoven fabric layer 61 in the around-leg gather 60, the use amount of a nonwoven fabric can be reduced. A tip portion of the around-leg gather 60 comes into contact with a skin. Therefore, by disposing the nonwoven fabric absent portion 65 so as to avoid the portion, the liquid impervious sheet 64 less likely to come into contact with a skin, and deterioration of a texture can be suppressed.

By extending the inner nonwoven fabric layer 61 to a side of the top sheet 30 in the embodiment illustrated in Figs. 1 to 6, or by forming the around-leg gather 60 of the structures illustrated in Figs. 7 and 8, the entire liquid impervious sheet 64 may be concealed.

The elastically stretchable gather member 63 may be disposed only in a tip portion of the around-leg gather 60. However, as in the illustrated embodiment, a plurality of the elastically stretchable gather members 63 is preferably disposed at intervals in a direction from a tip to a base of the around-leg gather 60. In a usual case, the number of the elastically stretchable gather members 63 is preferably 2 to 6, and a mutual interval therebetween is preferably 3 to 10 mm. In this way, by disposing a plurality of the elastically stretchable gather members 63 at intervals, a portion therebetween is recessed outward. Therefore, as in the illustrated embodiment, disposition of the nonwoven fabric absent portion 65 only in this interval portion is preferable because the liquid impervious sheet 64 exposed to the nonwoven fabric absent portion 65 is recessed and less likely to come into contact with a skin. In this case, as in the embodiment illustrated in Figs. 1 to 6, particularly preferably, one or more elastically stretchable gather members 63 are disposed at intervals at least only in a tip portion and a base portion of the around-leg gather 60, and the nonwoven fabric absent portion 65 is disposed only in an interval portion between the elastically stretchable gather member 63 at the base portion and the elastically stretchable gather member 63 at the tip portion.

A front-back direction range in which the elastically stretchable gather member 63 is disposed in the around-leg gather 60 can be the entire around-leg gather 60 in a front-back direction, but is preferably equal to or less than a front-back direction range of a rising portion.

As long as the elastically stretchable gather member 63 is disposed between the inner nonwoven fabric layer 61 and the outer nonwoven fabric layer 62 (not disposed in the nonwoven fabric absent portion 65 because of this), the elastically stretchable gather member 63 can be disposed inside the liquid impervious sheet 64 incorporated in the around-leg gather 60 as in the embodiments illustrated in Figs. 3 and 9, or can be disposed outside the liquid impervious sheet 64 as in the embodiment illustrated in Fig. 10.

A range in which the liquid pervious film 64 is disposed can be from a base to an intermediate position between the base and a tip as long as being in a range from the base to a position closer to the tip side than the base in the around-leg gather 60. However, in order to sufficiently improve a water shielding property, it is desirable that the liquid pervious film 64 is disposed up to the tip portion. In particular, as in the embodiment illustrated in Figs. 3 and 4, by disposing the liquid pervious film 64 up to a position slightly separated from the tip portion (for example, a distance corresponding to a plurality of the gather elastic members, specifically about 5 to 30 mm) and not incorporating the liquid pervious film 64 into the tip portion, softness of a texture is preferably secured.

In an embodiment in which the liquid impervious sheet 64 is exposed to the nonwoven fabric absent portion 65, in a portion 60W overlapping with the front outer body 12F and the back outer body 12B in the around-leg gather 60, the liquid impervious sheet 64 exposed to the nonwoven fabric absent portion 65 maybe pressed against a skin. However, as in the embodiment illustrated in Figs. 1 to 6, when the portion 60W is fixed to the front outer body 12F and the back outer body 12B and contracted in a width direction by the elastically stretchable members 15 and 19 of the front outer body 12F and the back outer body 12B, the contact area of the portion 60W to a skin is significantly reduced due to contraction wrinkles even if the liquid impervious sheet 64 is exposed, and therefore an influence on a texture is small. Incidentally, in the around-leg gather 60 in the present embodiment, a region between the fixed portions 60W with respect to the front outer body 12F and the back outer body 12B rises toward a periphery of a leg with a side edge of the absorber 56 as a base as illustrated by a two-dot chain line in Fig. 3 as the elastically stretchable gather member 63 contracts.

A member configuration of the around-leg gather 60 is not particularly limited, and a known structure can be adopted. In the embodiment illustrated in Figs. 1 to 6, the top sheet 30 is formed of a nonwoven fabric, and both sides of the top sheet 30 in a width direction are extended from a side edge of the absorber 56. In addition, a gather sheet 66 formed of a nonwoven fabric is disposed on a back surface side of the absorber 56, and both sides of the gather sheet 66 in a width direction are extended from the side edge of the absorber 56. Furthermore, a side end portion of the gather sheet 66 is folded back, and a tip of a folded-back portion 66r is separated from a tip of the top sheet 30. The liquid impervious sheet 64 is disposed at least from a portion between the folded-back portions 66r of the gather sheet 66 to a portion between the top sheet 30 and the gather sheet 66. As a result, the outer nonwoven fabric layer 62 is formed by a portion other than the folded-back portion 66r of the gather sheet 66. The inner nonwoven fabric layer 61 is formed by the folded-back portion 66r of the gather sheet 66 and a portion of the top sheet 30 extending to a lateral side of the absorber 56. The nonwoven fabric absent portion 65 is formed by a portion where the folded-back portion 66r of the gather sheet 66 and the top sheet 30 are separated from each other. In this way, by forming the inner nonwoven fabric layer 61 on a base side of the nonwoven fabric absent portion 65 in the around-leg gather 60 by the top sheet 30, and forming the other portions by the gather sheet 66, the nonwoven fabric absent portion 65 can be disposed without cutting a material, a structure thereof is very simple, and manufacture thereof is easy.

In this case, it is preferred that the liquid impervious sheet 64 of the around-leg gather 60 extends from one of the around-leg gathers 60 to the other of the leg gathers 60 through the back surface side of the absorber 56 as in the embodiment illustrated in Figs. 3 and 4 because not only a water shielding property of the around-leg gather 60 but also a water shielding property of the back surface side of the absorber 56 can be secured integrally. However, as in the embodiment illustrated in Figs. 7 and 8, the liquid pervious film 64 to be incorporated in the around-leg gather 60 and the liquid pervious film 11 covering the back surface side of the absorber 56 can be disposed separately from each other. In the latter case, a material of the liquid pervious film 64 to be incorporated in the around-leg gather 60 may be the same as or different from a material 11 of the liquid pervious film covering the back surface side of the absorber 56.

Similarly, as in the embodiment illustrated in Figs. 3 and 4, it is preferred that the gather sheet 66 is formed of an integral sheet from one of the around-leg gathers 60 to the other of the around-leg gathers 60 through the back surface side of the absorber 56 because a cloth-like outer surface can be obtained without separately disposing the above-described crotch portion cover sheet. However, as in the embodiment illustrated in Fig. 7, the gather sheet 66 and a crotch portion cover sheet 12M may be separately disposed.

As another structure of the around-leg gather 60, as in the embodiment illustrated in Figs. 7 and 8, it is also possible to adopt a structure including: an attachment portion 68 fixed to a back surface side of the inner body 200; an extension portion 69 going around a lateral side of the inner body 200 from the attachment portion 68 and extending to a side surface of the inner body 200; a fallen portion 69B formed by being fixed to the side surface of the inner body 200 while both end portions of the extension portion 69 in a front-back direction are in a fallen state; a free portion 69F formed by making an intermediate portion between the fallen portions in the extension portion non-fixed; and the elastically stretchable gather member 63 fixed in a stretched state in a front-back direction at least in a tip portion of the free portion 69F. In the around-leg gather 60, as the elastically stretchable gather member 63 contracts, the free portion 69F rises toward a periphery of a leg with a boundary between the free portion 69F and the attachment portion 68 as a base as illustrated by a two-dot chain line in Fig. 9.

The extension portion 69 of the around-leg gather 60 in the embodiment illustrated in Figs. 7 and 8 includes a root side portion toward the center in a width direction and a tip side portion folded back outward in the width direction from a tip of the root side portion. However, the extension portion 69 may include only the portion toward a center side in the width direction without being folded back outward in the width direction (not illustrated).

Meanwhile, in an intermediate region serving as a rising portion in the around-leg gather 60 in a front-back direction, at least one of a hot melt adhesive by various application methods and a fixing means by material welding such as heat sealing or ultrasonic sealing can be used for bonding the inner nonwoven fabric layer 61 to the outer nonwoven fabric layer 62 and fixing the elastically stretchable gather member 63 sandwiched therebetween. When the entire surfaces of the inner nonwoven fabric layer 61 and the outer nonwoven fabric layer 62 are bonded to each other, flexibility is impaired. Therefore, preferably, a portion other than a bonded portion of the elastically stretchable gather member 63 is not bonded or weakly bonded. In the illustrated embodiment, by applying a hot melt adhesive only to an outer peripheral surface of the elastically stretchable gather member 63 by an application means such as a comb gun or a SureWrap nozzle, and sandwiching the elastically stretchable gather member 63 between the inner nonwoven fabric layer 61 and the outer nonwoven fabric layer 62, an elongated elastically stretchable member is fixed to the inner nonwoven fabric layer 61 and the outer nonwoven fabric layer 62 only with the hot melt adhesive applied to the outer peripheral surface of the elastically stretchable gather member 63, and the inner nonwoven fabric layer 61 and the outer nonwoven fabric layer 62 are fixed to each other.

In non-rising portions on both sides of the around-leg gather 60 in a front-back direction, at least one of a hot melt adhesive by various application methods and a fixing means 67 by material welding such as heat sealing or ultrasonic sealing can be used for sticking the inner nonwoven fabric layer 61 to the outer nonwoven fabric layer 62, fixing the around-leg gather 60 in the embodiment illustrated in Figs. 1 to 6 to the front outer body 12F and the back outer body 12B, fixing the root side portion and the tip side portion in the around-leg gather 60 in the embodiment illustrated in Figs. 7 and 8, and fixing the root side portion and the tip side portion to a side surface of the inner body 200. In the illustrated embodiment, the hot melt adhesive and the fixing means 67 by material welding are combined, but these can also be fixed by only one of the means.

The size of the around-leg gather 60 can be determined appropriately. However, in a case of a baby disposable diaper, the erect height of the around-leg gather 60 (interval in a width direction between a tip and a base in an unfolded state) is preferably 15 to 60 mm, and particularly preferably 20 to 40 mm.

In each of the above-described embodiments, as the inner nonwoven fabric layer 61 and the outer nonwoven fabric layer 62, a product obtained by subjecting a soft nonwoven fabric having excellent uniformity and concealability, such as a spunbonded nonwoven fabric (SS, SSS, or the like), an SMS nonwoven fabric (SMS, SSMMS, or the like), or a melt blown nonwoven fabric to a water repellent treatment with silicon or the like as necessary can be used suitably. The nonwoven fabric preferably has a fiber basis weight of about 10 to 30 g/m². In the embodiment illustrated in Figs. 3 and 4, as can be seen from the fact that the inner nonwoven fabric layer 61 closer to a base side than the nonwoven fabric absent portion 65 is formed by the top sheet 30, it is also possible to make a material of each of the inner nonwoven fabric layer 61 and the outer nonwoven fabric layer 62 partially different, or it is also possible to make the materials of the inner nonwoven fabric layer 61 and the outer nonwoven fabric layer 62 different from each other.

In each of the above-described embodiments, as the elastically stretchable gather member 63, an elongated elastically stretchable member such as a thread-shaped rubber or a belt-shaped rubber can be used. In a case where a rubber thread is used, the rubber thread preferably has a thickness of 470 to 1240 dtex, and more preferably has a thickness of 620 to 940 dtex. The rubber thread preferably has a stretch rate of 150 to 350%, and more preferably has a stretch rate of 200 to 300% at the time of fixing.

In each of the above-described embodiments, one row of the around-leg gather 60 is disposed on each of the right and left. However, a plurality of rows thereof can also be disposed.

### <Explanation of terms in specification>

In a case where the following terms are used in the specification, the terms have the following meanings unless otherwise specified in the specification.
· "Front-back (longitudinal) direction" means a direction connecting a ventral side (front side) and a dorsal side (back side), and "width direction" means a direction orthogonal to the front-back direction (horizontal direction).
· "MD" in a manufacturing method means a machine direction, that is, a flow direction (transfer direction) of a manufacturing line, and the "CD" means a lateral direction orthogonal to the MD. The MD in a product (absorbent article) is a direction of fiber orientation of a nonwoven fabric. The fiber orientation is a direction in which fibers of a nonwoven fabric are aligned, and can be determined by, for example, a measurement method in accordance with a fiber orientation test method based on zero distance tensile strength by the TAPPI standard method T481 or a simple measurement method for determining a fiber orientation direction based on a tensile strength ratio in a front-back direction and a width direction. In a usual case, one of the MD and the CD is a front-back direction, and the other is a width direction. In most products, the front-back direction is the MD, and the width direction is the CD.
· "Unfolded state" means a flatly unfolded state without contraction or slackness.
· "Stretch rate" means a value obtained when a natural length is 100%.
· "Artificial urine" is a mixture of 2% by weight of urea, 0.8% by weight of sodium chloride, 0.03% by weight of calcium chloride dihydrate, 0.08% by weight of magnesium sulfate heptahydrate, and 97.09% by weight of deionized water, and is used at a temperature of 40°C unless otherwise specified.
· "Gel strength" is measured as follows. To 49.0 g of artificial urine, 1.0 g of super absorbent polymer is added, and the resulting mixture is stirred with a stirrer. The gel thus generated is left in a thermohygrostat at 40°C × 60%RH for three hours. Thereafter, the temperature is returned to room temperature, and gel strength is measured with a curdmeter (Curdmeter-MAXME-500 manufactured by I. Techno Engineering Co., Ltd.) .
· "Basis weight" is measured as follows. A sample or a test piece is predried and then left in a test chamber or an apparatus in a standard state (test location is at a temperature of 20 ± 5°C and a relative humidity of 65% or less) so as to have a constant weight. Predrying refers to causing a sample or a test piece to have a constant weight in an environment that a relative humidity is 10 to 25% and a temperature does not exceed 50°C. Incidentally, fibers having an official moisture regain of 0.0% do not have to be predried. A sample of 200 mm × 250 mm (± 2 mm) in size is cut out from a test piece having a constant weight using a cutting template (200 mm × 250 mm, ± 2 mm) . The weight of the sample is measured. The weight is multiplied by 20 to calculate the weight per square meter to be used as a basis weight.
· "Thickness" is automatically measured under conditions that a load is 10 gf/cm² and a pressing area is 2 cm² using an automatic thickness meter (KES-G5 handy compression testing machine).
· "Water absorption capacity" is measured in accordance with JIS K7223-1996 "Test method for water absorption capacity of super absorbent polymer".
· "Water absorption rate" is "time to end point" when JIS K7224-1996 "Test method for water absorption rate of super absorbent polymer" is performed using 2 g of super absorbent polymer and 50 g of physiological saline.
· "Bending resistance" means "8.21.1 A method (45° cantilever method)" of JIS L 1096: 2010 "Testing methods for woven and knitted fabrics".
· "Melt viscosity" is measured at a temperature of 140 °C using a Brookfield B-type viscometer (spindle No. 027) in accordance with JIS Z 8803.
· "Air permeability" is a value obtained by measuring time in which 300 ml of gas passes through a target material in accordance with "Gurley tester method" of JIS P 8117.
· In a case where environmental conditions in a test and a measurement are not described, the test and the measurement are performed in a test room or an apparatus in a standard state (test location is at a temperature of 20 ± 5°C and a relative humidity of 65% or less).
· The size of each portion means a size not in a natural length state but in an unfolded state unless otherwise specified.

### Industrial Applicability

The present invention is suitable for an underpants-type disposable diaper as in the above example, but can be applied to a general absorbent article such as a sanitary napkin as well as a tape-type or a pad-type disposable diaper.

### Reference Signs List

- 11: Liquid impervious sheet
- 12A: Side seal portion
- 12B: Back outer body
- 12F, 12B: Outer body
- 12F: Front outer body
- 12H: Inner sheet layer
- 12S: Outer sheet layer
- 15, 18: Under-waist portion elastically stretchable member
- 16: Cover portion elastically stretchable member
- 17: Waist portion elastically stretchable member
- 19: Elastically stretchable member
- 200: Inner body
- 30: Top sheet
- 40: Intermediate sheet
- 50: Absorbent element
- 51: Upper accumulation layer
- 52: Lower accumulation layer
- 53: Reinforcing layer
- 54: Narrower portion
- 56: Absorber
- 56P: Super absorbent polymer particles
- 56s: Short fiber sheet
- 58: Wrapping sheet
- 58c: Connecting portion
- 60: Leg gather
- 61: Inner nonwoven fabric layer
- 62: Outer nonwoven fabric layer
- 63: Elastically stretchable gather member
- 64: Liquid impervious sheet
- 65: Nonwoven fabric absent portion
- 66: Gather sheet
- 66r: Folded-back portion
- 100: Manufacturing equipment
- 101: Defibrating machine
- 102: Fiber-stacking drum
- 102m: Absorber mold
- 102a, 102b: Supply chamber
- 102a: First chamber
- 102b: Second chamber
- 102i: Inlet
- 102x: Outlet
- G1: First adhesive layer
- G2: Second adhesive layer
- G3: Third adhesive layer
- H1, H2: Hot melt adhesive
- 71: First accumulation layer
- 72: Second accumulation layer

## Claims

1. A method for manufacturing an absorbent article including an absorbent element (50),
using a continuous body of the absorbent element (50) manufactured
with a fiber accumulating apparatus which
includes a fiber accumulating drum (102), which is rotatably driven and has a recessed absorber mold (102m) formed on an outer peripheral surface thereof, a front-back direction of the recessed absorber mold (102m) being a rotational direction of the drum and a bottom surface of the absorber mold (102m) being provided with many suction holes formed thereon, and
flies and supplies an accumulation material to the outer peripheral surface of the fiber accumulating drum (102) on an air flow by suction from the suction holes, and accumulates the accumulation material in the absorber mold to form an absorber (56),
by releasing the absorber (56) from the absorber mold (102m) and transferring the released absorber (56) onto the wrapping sheet (58) when the absorber (56) formed in the absorber mold (102m)is located so as to face a continuous belt-shaped wrapping sheet (58) supplied along the outer peripheral surface of the fiber accumulating drum (102) by rotation of the fiber accumulating drum (102), wherein a first adhesive layer (G1) is formed in advance on the transfer surface of the wrapping sheet (58), to which the absorber (56) is transferred by applying a hot melt adhesive (M1), and the absorber (56) is bonded to the wrapping sheet (58) with the first adhesive layer (G1), and
after the absorber (56) is transferred onto the wrapping sheet (58), a hot melt adhesive (M2) is applied over the maximum width of an upper surface of the absorber (56) to form a second adhesive layer (G2),
after that, by folding back both side portions (58s) projecting from both sides of the absorber (56) in the wrapping sheet (58) in a CD (Cross Direction) at positions along both side edges of the absorber (56) and bonding said side portions (58s) to an upper surface of the absorber (56) with both end portions in the CD overlapped with each other, and bonded with a third adhesive layer (G3) formed by applying a hot melt adhesive (M3) in advance to the overlapping portion of side portions (58s) to form a connecting portion (58c), such that the continuous body of the absorbent element (50) in which absorbers (56) are intermittently fixed in a MD (Machine Direction) is formed in the cylindrical continuous body of the wrapping sheet (58) continuous in the MD, and the method comprising:
accumulating the accumulation material in the absorber mold (102m) at a first accumulation position and at a second accumulation position disposed in this order on the outer peripheral surface of the fiber accumulating drum (102) at intervals in the rotational direction of the fiber accumulating drum (102);
supplying a continuous belt-shaped air-permeable reinforcing layer (53) continuous in the rotational direction of the fiber accumulating drum (102) so as to pass over the absorber mold (102m) on the outer peripheral surface of the fiber accumulating drum (102) between the first accumulation position and the second accumulation position;
forming sequentially the absorbers (56) each, in the absorber mold, including a first accumulation layer (71) accumulated at the first accumulation position, a second accumulation layer (72) accumulated at the second accumulation position, and a portion of the continuous belt-shaped reinforcing layer (53), the portion being directly sandwiched between the first accumulation layer (71) and the second accumulation layer (72); and
transferring the continuous belt-shaped reinforcing layer (53) and the absorber (56) onto the continuous belt-shaped wrapping sheet (58) from the fiber accumulating drum (102).

2. The method for manufacturing an absorbent article according to claim 1, comprising:
accumulating an accumulation material containing short fibers and not containing super absorbent polymer particles (56P) at the first accumulation position; and
accumulating an accumulation material containing short fibers and super absorbent polymer particles (56P) at the second accumulation position.

3. The method for manufacturing an absorbent article according to claim 2, wherein the reinforcing layer (53) is formed of a nonwoven fabric.

4. The method for manufacturing an absorbent article according to any one of claims 1 to 3, wherein
the absorber mold (102m) has a narrower portion (54) narrowed on an axial direction central side in an intermediate portion in the rotational direction, and
a width of the reinforcing layer (53) is narrower than a width of a narrowest portion of the narrower portion (54), and the reinforcing layer (53) is stacked so as to pass through the narrowest portion of the narrower portion (54).

5. An absorbent article comprising an absorbent element (50) including an absorber (56) for absorbing excrement and a wrapping sheet (58) wound around the absorber (56) cylindrically so as to surround front and back surfaces and both sides surfaces of the absorber (56), wherein
the wrapping sheet (58) has an intermediate portion (58m) located on one side of the front and back of the absorber (56), both side portions (58s) folded back from the intermediate portion (58m) to the other side of the absorber (56), and a connecting portion (58c) formed by overlapping tip portions of both side portions (58s) of the wrapping sheet (58) on the other side of the absorber (56), and including a first adhesive layer (G1) bonding the intermediate portion (58m), the absorber (56) and a second adhesive layer (G2) bonding the both side portions (58s) of the wrapping sheet (58)and the absorber (56), and a third adhesive layer (G3) bonding the overlapping tip portions of both side portions (58s) of the wrapping sheet (58) to form the connecting portion (58c), wherein
the absorber (56) includes: a reinforcing layer (53) continuous in the front-back direction; an upper accumulation layer (51) which is in contact with an upper surface of the reinforcing layer (53) and in which an accumulation material is accumulated; and a lower accumulation layer (52) which is in contact with a lower surface of the reinforcing layer (53) and in which an accumulation material is accumulated,
a length of the reinforcing layer (53) in the front-back direction is equal to a length of the absorbent element (50) in the front-back direction, and
front and back end portions of the wrapping sheet (58) project from the front and back of the absorber (56).

6. The absorbent article according to claim 5, wherein the reinforcing layer (53) is formed of a nonwoven fabric or a filament assembly.

7. The absorbent article according to claim 5 or 6, wherein
the upper accumulation layer (51) and the lower accumulation layer (52) contain short fibers as an accumulation material,
at least one of the upper accumulation layer (51) and the lower accumulation layer (52) contains super absorbent polymer particles (56P) as an accumulation material,
a weight of the super absorbent polymer particles (56P) is larger than a weight of fibers contained in the upper accumulation layer (51) and the lower accumulation layer (52), and
a content of the super absorbent polymer particles (56P) stepwise or continuously increases from a back surface side to a front surface side.

8. The absorbent article according to any one of claims 5 to 7, wherein
each of the upper accumulation layer (51) and the lower accumulation layer (52) has a narrower portion (54) narrowed on a width direction center side in an intermediate portion in the front-back direction, and
the reinforcing layer (53) is disposed within a width direction range of a narrowest portion of the narrower portion (54) .

9. The absorbent article according to claim 5 or 6, wherein
at least one of the upper accumulation layer (51) and the lower accumulation layer (52) contains super absorbent polymer particles (56P) as an accumulation material,
the reinforcing layer (53) is formed of a nonwoven fabric, and an amount of super absorbent polymer particles (56P) in a region including the reinforcing layer (53) in a thickness direction is largest.

## Patentansprüche

1. Verfahren zur Herstellung eines absorbierenden Artikels mit einem absorbierenden Element (50),
Verwenden eines kontinuierlichen Körpers des absorbierenden Elements (50) hergestellt mit einer Fasersammelvorrichtung, die
eine Fasersammeltrommel (102) umfasst, die rotierbar angetrieben wird und eine vertiefte Absorberform (102m) aufweist, die an ihrer äußeren Umfangsfläche ausgebildet ist, wobei eine Vorwärts-Rückwärts-Richtung der vertieften Absorberform (102m) eine Rotationsrichtung der Trommel ist und eine Bodenfläche der Absorberform (102m) mit vielen darauf ausgebildeten Sauglöchern versehen ist, und
der äußeren Umfangsfläche der Fasersammeltrommel (102) durch einen Luftstrom durch Ansaugen aus den Ansauglöchern ein Akkumulationsmaterial fliegt und zuführt bzw. fliegend zuführt und das Akkumulationsmaterial in der Absorberform zur Bildung eines Absorbers (56) anhäuft,
durch Freigeben des Absorbers (56) aus der Absorberform (102m) und Übertragen des freigegebenen Absorbers (56) auf eine Umhüllungslage (58), wenn der in der Absorberform (102m) gebildete Absorber (56) so angeordnet ist, dass er der kontinuierlichen bandförmigen Umhüllungslage (58) gegenüberliegt, die entlang der äußeren Umfangsfläche der Fasersammeltrommel (102) durch Rotation der Fasersammeltrommel (102) zugeführt wird, wobei eine erste Klebstoffschicht (G1) im Voraus auf der Übertragungsoberfläche der Umhüllungslage (58) gebildet wird, auf die der Absorber (56) durch Aufbringen eines Heißschmelzklebstoffs (M1) übertragen wird, und der Absorber (56) mit der ersten Klebstoffschicht (G1) an die Umhüllungslage (58) gebunden wird, und
nachdem der Absorber (56) auf die Umhüllungslage (58) übertragen wurde, ein Heißschmelzkleber (M2) über die maximale Breite einer oberen Fläche des Absorbers (56) aufgetragen wird, um eine zweite Klebeschicht (G2) zu bilden,
danach durch Zurückfalten beider Seitenabschnitte (58s), die von beiden Seiten des Absorbers (56) in der Umhüllungslage (58) in einer CD (Querrichtung (Cross Direction)) an Positionen entlang beider Seitenkanten des Absorbers (56) vorstehen, und Verbinden der Seitenabschnitte (58s) mit einer oberen Fläche des Absorbers (56), wobei beide Endabschnitte in der CD einander überlappen, und mit einer dritten Klebstoffschicht (G3) verbunden werden, die durch Auftragen eines Heißschmelzklebstoffs (M3) im Voraus auf den überlappenden Abschnitt der Seitenabschnitte (58s) gebildet wird, um einen Verbindungsabschnitt (58c) zu bilden, so dass der kontinuierliche Körper des absorbierenden Elements (50), in dem Absorber (56) intermittierend in einer MD (Maschinenrichtung) befestigt sind, in dem zylindrischen kontinuierlichen Körper der in der MD kontinuierlichen Umhüllungslage (58) gebildet wird, und wobei das Verfahren umfasst:
Anhäufen des Akkumulationsmaterials in der Absorberform (102m) an einer ersten Akkumulationsposition und an einer zweiten Akkumulationsposition, die in dieser Reihenfolge auf der äußeren Umfangsfläche der Fasersammeltrommel (102) in Intervallen in der Rotationsrichtung der Fasersammeltrommel (102) angeordnet sind;
Zuführen einer kontinuierlichen, gürtelförmigen, luftdurchlässigen Verstärkungsschicht (53), die in der Rotationsrichtung der Fasersammeltrommel (102) kontinuierlich ist, so dass sie über die Absorberform (102m) auf der äußeren Umfangsfläche der Fasersammeltrommel (102) zwischen der ersten Akkumulationsposition und der zweiten Akkumulationsposition verläuft;
sequentielles Ausbilden der Absorber (56), von denen jeder in der Absorberform eine erste Akkumulationsschicht (71), die an der ersten Akkumulationsposition akkumuliert ist, eine zweite Akkumulationsschicht (72), die an der zweiten Akkumulationsposition akkumuliert ist, und einen Abschnitt der kontinuierlichen bandförmigen Verstärkungsschicht (53) umfasst, wobei der Abschnitt direkt zwischen der ersten Akkumulationsschicht (71) und der zweiten Akkumulationsschicht (72) sandwichartig angeordnet ist; und
Übertragen der kontinuierlichen bandförmigen Verstärkungsschicht (53) und des Absorbers (56) auf die kontinuierliche bandförmige Umhüllungslage (58) von der Fasersammeltrommel (102).

2. Verfahren zur Herstellung eines absorbierenden Artikels nach Anspruch 1, umfassend:
Akkumulieren eines Akkumulationsmaterials, das kurze Fasern enthält und keine superabsorbierenden Polymerpartikel (56P) enthält, an der ersten Akkumulationsposition; und
Akkumulieren eines Akkumulationsmaterials, das kurze Fasern und superabsorbierende Polymerpartikel (56P) enthält, an der zweiten Akkumulationsposition.

3. Verfahren zur Herstellung eines absorbierenden Artikels nach Anspruch 2, wobei die Verstärkungsschicht (53) aus einem Vliesstoff gebildet ist.

4. Verfahren zur Herstellung eines absorbierenden Artikels nach einem der Ansprüche 1 bis 3, wobei
die Absorberform (102m) einen schmaleren Abschnitt (54) aufweist, der an einer zentralen Seite in axialer Richtung in einem Zwischenabschnitt in der Rotationsrichtung verengt ist, und
eine Breite der Verstärkungsschicht (53) schmaler ist als eine Breite eines engsten Abschnitts des schmaleren Abschnitts (54), und die Verstärkungsschicht (53) so gestapelt ist, dass sie durch den engsten Abschnitt des schmaleren Abschnitts (54) hindurchgelangt.

5. Absorbierender Artikel mit einem absorbierenden Element (50), das einen Absorber (56) zum Absorbieren von Exkrementen und eine Umhüllungslage (58) aufweist, die zylindrisch um den Absorber (56) gewickelt ist, so dass sie die vorderen und hinteren Oberflächen und beide seitlichen Oberflächen des Absorbers (56) umgibt, wobei
die Umhüllungslage (58) einen Zwischenabschnitt (58m), der auf einer Seite der Vorderseite und Rückseite des Absorbers (56) angeordnet ist, beide Seitenabschnitte (58s), die von dem Zwischenabschnitt (58m) zu der anderen Seite des Absorbers (56) zurückgefaltet sind, und einen Verbindungsabschnitt (58c) aufweist, der durch Überlappen von Spitzenabschnitten der beiden Seitenabschnitte (58s) der Umhüllungslage (58) auf der anderen Seite des Absorbers (56) gebildet ist, und mit einer ersten Klebstoffschicht (G1), die den Zwischenabschnitt (58m) bindet, dem Absorber (56) und einer zweiten Klebstoffschicht (G2), die die beiden Seitenabschnitte (58s) der Umhüllungslage (58) und den Absorber (56) verbindet, und einer dritten Klebstoffschicht (G3), die die überlappenden Spitzenabschnitte der beiden Seitenabschnitte (58s) der Umhüllungslage (58) verbindet, um den Verbindungsabschnitt (58c) zu bilden, wobei
der Absorber (56) umfasst: eine Verstärkungsschicht (53), die in der Vorwärts-Rückwärts-Richtung kontinuierlich ist; eine obere Akkumulationsschicht (51), die in Kontakt mit einer oberen Oberfläche der Verstärkungsschicht (53) ist und in der ein Akkumulationsmaterial akkumuliert wird; und eine untere Akkumulationsschicht (52), die in Kontakt mit einer unteren Oberfläche der Verstärkungsschicht (53) ist und in der ein Akkumulationsmaterial akkumuliert wird,
eine Länge der Verstärkungsschicht (53) in der Vorwärts-Rückwärts-Richtung gleich einer Länge des absorbierenden Elements (50) in der Vorwärts-Rückwärts-Richtung ist, und
die vorderen und hinteren Endabschnitte der Umhüllungslage (58) von der Vorderseite und Rückseite des Absorbers (56) vorstehen.

6. Absorbierender Artikel nach Anspruch 5, wobei die Verstärkungsschicht (53) aus einem Vliesstoff oder einer Filamentanordnung gebildet ist.

7. Absorbierender Artikel nach Anspruch 5 oder 6, wobei
die obere Akkumulationsschicht (51) und die untere Akkumulationsschicht (52) kurze Fasern als Akkumulationsmaterial enthalten,
mindestens eine der oberen Akkumulationsschicht (51) und der unteren Akkumulationsschicht (52) superabsorbierende Polymerpartikel (56P) als Akkumulationsmaterial enthält,
ein Gewicht der superabsorbierenden Polymerteilchen (56P) größer ist als ein Gewicht der in der oberen Akkumulationsschicht (51) und der unteren Akkumulationsschicht (52) enthaltenen Fasern, und
ein Gehalt an superabsorbierenden Polymerpartikeln (56P) schrittweise oder kontinuierlich von der Rückseite zur Vorderseite zunimmt.

8. Absorbierender Artikel nach einem der Ansprüche 5 bis 7, wobei
die obere Akkumulationsschicht (51) und die untere Akkumulationsschicht (52) jeweils einen schmaleren Abschnitt (54) aufweisen, der an einer Mittelseite in Breitenrichtung in einem Zwischenabschnitt in der Vorwärts-Rückwärts-Richtung verengt ist, und
die Verstärkungsschicht (53) innerhalb eines Breitenrichtungsbereichs eines schmalsten Abschnitts des schmaleren Abschnitts (54) angeordnet ist.

9. Absorbierender Artikel nach Anspruch 5 oder 6, wobei
mindestens eine der oberen Akkumulationsschicht (51) und der unteren Akkumulationsschicht (52) superabsorbierende Polymerpartikel (56P) als ein Akkumulationsmaterial enthält,
die Verstärkungsschicht (53) aus einem Vliesstoff gebildet ist, und eine Menge an superabsorbierenden Polymerteilchen (56P) in einem Bereich, der die Verstärkungsschicht (53) einschließt, in einer Dickenrichtung am größten ist.

## Revendications

1. Procédé de fabrication d'un article absorbant comprenant un élément absorbant (50),
utilisant un corps continu de l'élément absorbant (50) fabriqué avec un appareil d'accumulation de fibres qui
comprend un tambour d'accumulation de fibres (102), qui est entraîné en rotation et a un moule d'absorbeur encastré (102m) formé sur une surface périphérique externe de celui-ci, une direction avant-arrière du moule d'absorbeur encastré (102m) étant une direction de rotation du tambour et une surface inférieure du moule d'absorbeur (102m) étant dotée de nombreux trous d'aspiration formés dessus, et
fait voler et fournit un matériau d'accumulation à la surface périphérique externe du tambour d'accumulation de fibres (102) sur un flux d'air par aspiration à partir des trous d'aspiration, et accumule le matériau d'accumulation dans le moule d'absorbeur pour former un absorbeur (56),
en libérant l'absorbeur (56) du moule d'absorbeur (102m) et en transférant l'absorbeur libéré (56) sur la feuille d'emballage (58) lorsque l'absorbeur (56) formé dans le moule d'absorbeur (102m) est situé de sorte qu'il soit face à une feuille d'emballage en forme de courroie continue (58) fournie le long de la surface périphérique externe du tambour d'accumulation de fibres (102) par rotation du tambour d'accumulation de fibres (102), une première couche adhésive (G1) étant formée à l'avance sur la surface de transfert de la feuille d'emballage (58), sur laquelle l'absorbeur (56) est transféré par application d'un adhésif thermofusible (M1), et l'absorbeur (56) est lié à la feuille d'emballage (58) avec la première couche adhésive (G1), et
après que l'absorbeur (56) soit transféré sur la feuille d'emballage (58), un adhésif thermofusible (M2) est appliqué sur la largeur maximale d'une surface supérieure de l'absorbeur (56) pour former une seconde couche adhésive (G2),
après quoi, en repliant les deux parties latérales (58s) faisant saillie des deux côtés de l'absorbeur (56) dans la feuille d'emballage (58) dans une direction transversale (CD) à des positions le long des deux bords latéraux de l'absorbeur (56) et en liant lesdites parties latérales (58s) sur une surface supérieure de l'absorbeur (56), les deux parties d'extrémité de la direction transversale (CD) se superposant l'une avec l'autre et étant liées avec une troisième couche adhésive (G3) formée en appliquant un adhésif thermofusible (M3) à l'avance sur la partie superposée des parties latérales (58s) pour former une partie de liaison (58c), de telle sorte que le corps continu de l'élément absorbant (50) dans lequel les absorbeurs (56) sont fixés par intermittence dans un sens machine (MD) est formé dans le corps continu cylindrique de la feuille d'emballage (58) continue dans le sens machine (MD), et le procédé comprenant :
l'accumulation du matériau d'accumulation dans le moule d'absorbeur (102m) au niveau d'une première position d'accumulation et d'une seconde position d'accumulation disposées dans cet ordre sur la surface périphérique externe du tambour d'accumulation de fibres (102) à intervalles dans le sens de rotation du tambour d'accumulation de fibres (102);
la fourniture d'une couche de renforcement perméable à l'air en forme de courroie continue (53) continue dans le sens de rotation du tambour d'accumulation de fibres (102) de manière à passer sur le moule d'absorbeur (102m) sur la surface périphérique externe du tambour d'accumulation de fibres (102) entre la première position d'accumulation et la deuxième position d'accumulation ;
la formation séquentielle des absorbeurs (56) chacun, dans le moule d'absorbeur, comprenant une première couche d'accumulation (71) accumulée au niveau de la première position d'accumulation, une seconde couche d'accumulation (72) accumulée au niveau de la seconde position d'accumulation, et une partie de la couche de renforcement en forme de courroie continue (53), la partie étant directement intercalée entre la première couche d'accumulation (71) et la seconde couche d'accumulation (72); et
le transfert de la couche de renforcement en forme de courroie continue (53) et de l'absorbeur (56) sur la feuille d'emballage en forme de courroie continue (58) à partir du tambour d'accumulation de fibres (102).

2. Procédé de fabrication d'un article absorbant selon la revendication 1, comprenant :
l'accumulation d'un matériau d'accumulation contenant des fibres courtes et ne contenant pas de particules polymères super absorbantes (56P) au niveau de la première position d'accumulation ; et
l'accumulation d'un matériau d'accumulation contenant des fibres courtes et des particules polymères super absorbantes (56P) au niveau de la seconde position d'accumulation.

3. Procédé de fabrication d'un article absorbant selon la revendication 2, dans lequel la couche de renforcement (53) est formée d'un tissu non tissé.

4. Procédé de fabrication d'un article absorbant selon l'une des revendications 1 à 3, dans lequel
le moule d'absorbeur (102m) présente une partie plus étroite (54) rétrécie sur un côté central dans la direction axiale dans une partie intermédiaire dans la direction de rotation, et
une largeur de la couche de renforcement (53) est plus étroite qu'une largeur d'une partie la plus étroite de la partie la plus étroite (54), et la couche de renforcement (53) est empilée de manière à passer à travers la partie la plus étroite de la partie la plus étroite (54).

5. Article absorbant comprenant un élément absorbant (50) comprenant un absorbeur (56) pour absorber les excréments et une feuille d'emballage (58) enroulée autour de l'absorbeur (56) de manière cylindrique de manière à entourer les surfaces avant et arrière et les surfaces des deux côtés de l'absorbeur (56), dans lequel
la feuille d'emballage (58) comporte une partie intermédiaire (58m) située sur un côté de l'avant et de l'arrière de l'absorbeur (56), les deux parties latérales (58s) étant repliées depuis la partie intermédiaire (58m) vers l'autre côté de l'absorbeur (56), et une partie de liaison (58c) formée par le chevauchement des parties de pointe des deux parties latérales (58s) de la feuille d'emballage (58) de l'autre côté de l'absorbeur (56), et comprenant une première couche adhésive (G1) liant la partie intermédiaire (58m), l'absorbeur (56) et une deuxième couche adhésive (G2) liant les deux parties latérales (58s) de la feuille d'emballage (58) et l'absorbeur (56), et une troisième couche adhésive (G3) liant les parties de pointe se chevauchant des deux parties latérales (58s) de la feuille d'emballage (58) pour former la partie de liaison (58c), dans lequel
l'absorbeur (56) comprend : une couche de renforcement (53) continue dans la direction avant-arrière ; une couche d'accumulation supérieure (51) qui est en contact avec une surface supérieure de la couche de renforcement (53) et dans laquelle un matériau d'accumulation est accumulé; et une couche d'accumulation inférieure (52) qui est en contact avec une surface inférieure de la couche de renforcement (53) et dans laquelle un matériau d'accumulation est accumulé,
une longueur de la couche de renforcement (53) dans la direction avant-arrière est égale à une longueur de l'élément absorbant (50) dans la direction avant-arrière, et
les parties d'extrémité avant et arrière de la feuille d'emballage (58) font saillie depuis l'avant et l'arrière de l'absorbeur (56).

6. Article absorbant selon la revendication 5, dans lequel la couche de renforcement (53) est formée d'un tissu non tissé ou d'un assemblage de filaments.

7. Article absorbant selon la revendication 5 ou 6, dans lequel la couche d'accumulation supérieure (51) et la couche d'accumulation inférieure (52) contiennent des fibres courtes comme matériau d'accumulation,
au moins l'une parmi la couche d'accumulation supérieure (51) et la couche d'accumulation inférieure (52) contient des particules polymères super absorbantes (56P) en tant que matériau d'accumulation,
un poids des particules polymères super absorbantes (56P) est supérieur au poids des fibres contenues dans la couche d'accumulation supérieure (51) et la couche d'accumulation inférieure (52), et
une teneur en particules polymères super absorbantes (56P) augmente progressivement ou de manière continue depuis un côté surface arrière jusqu'à un côté surface avant.

8. Article absorbant selon l'une des revendications 5 à 7, dans lequel
chacune de la couche d'accumulation supérieure (51) et de la couche d'accumulation inférieure (52) a une partie plus étroite (54) rétrécie sur un côté central dans le sens de la largeur dans une partie intermédiaire dans la direction avant-arrière, et
la couche de renforcement (53) est disposée dans une plage de direction de largeur d'une partie la plus étroite de la partie la plus étroite (54) .

9. Article absorbant selon la revendication 5 ou 6, dans lequel au moins l'une parmi la couche d'accumulation supérieure (51) et la couche d'accumulation inférieure (52) contient des particules de polymère super absorbantes (56P) comme matériau d'accumulation, la couche de renforcement (53) est formée d'un tissu non tissé, et la quantité de particules polymères super absorbantes (56P) dans une région comprenant la couche de renforcement (53) dans le sens de l'épaisseur est la plus grande.
